# EUROPEAN PATENT SPECIFICATION

(11) **EP 1 960 777 B1**
(45) Date of publication and mention of the grant of the patent: **21.10.2009**
(21) Application number: 06838470.0
(22) Date of filing: 27.11.2006
(51) Int. Cl.: G01N 33/50, G01N 33/52, G01N 33/68, C12N 5/00, G01N 33/573

(54) **SERUM-FREE EXPANSION OF CELLS IN CULTURE**
SERUMFREIE EXPANSION VON ZELLEN IN KULTUR
EXPANSION SANS SERUM DE CELLULES EN CULTURE

(30) Priority: 28.11.2005 US 740173 P
(43) Date of publication of application: 27.08.2008
(73) Proprietor: Choongwae Pharma Corporation, Seoul 156-757 (KR)
(72) Inventor: KAHN, Michael, Keck School of Medicine at USC, Centre for Stem Cell and Regenerative Medicine, Los Angeles,CA 90033 (US)
(74) Representative: Crease, Devanand John
(86) International application number: PCT/US2006/045515
(87) International publication number: WO 2007/062243

(56) References cited:
- WO-A-03/104442
- US-A1- 2005 153 941
- RUEDIGER RALF ET AL: "Alterations in protein phosphatase 2A subunit interaction in human carcinomas of the lung and colon with mutations in the Abeta subunit gene" ONCOGENE, vol. 20, no. 15, 5 April 2001 (2001-04-05), pages 1892-1899, XP002426557 ISSN: 0950-9232
- CREYGHTON MENNO P ET AL: "PR72, a novel regulator of Wnt signaling required for naked cuticle function" GENES & DEVELOPMENT, vol. 19, no. 3, 1 February 2005 (2005-02-01), pages 376-386, XP002426558 ISSN: 0890-9369
- MCMILLAN ET AL: "Investigating Wnt signaling: a chemogenomic safari" DDT - DRUG DISCOVERY TODAY, ELSEVIER SCIENCE LTD, GB, vol. 10, no. 21, 1 November 2005 (2005-11-01), pages 1467-1474, XP005124585 ISSN: 1359-6446
- DATABASE Geneseq [Online] 2 June 2003 (2003-06-02), "Human DITHP intracellular signalling protein." XP002426565 retrieved from EBI accession no. GSP:ABR41330 Database accession no. ABR41330

## Description

### Field of the Invention

The present invention relates to compounds and methods for identifying compounds capable of promoting proliferation and preventing differentiation of stem/progenitor cells.

### Description of the Related Art

Stem cells have received significant interest over the last few years due to their potential, under suitable cellular microenvironments, to differentiate and develop into a wide array of cell and tissue types. Several important biomedical applications would be enabled by the ability to generate sufficient pools of adult stem cells, including cell replacement therapy, gene therapy, and tissue engineering. According to the National Institutes of Health, the therapeutic use of stem cells will become a cornerstone of medicine within the next two decades:
Given the enormous potential of stem cells to the development of new therapies for the most devastating diseases, when a readily available source of stem cells is identified, it is not too unrealistic to say that this research will revolutionize the practice of medicine and improve the quality and length of life (National Institutes of Health. Stem Cells: Scientific Progress and Future Research Directions. June 17, 2001).

However, the development of such applications for adult stem cells has been severely impaired due to the inability to propagate and expand functional adult stem cells in culture. To date, this has proven to be a singular challenge in stem cell research (Sherley, J. (2002) Stem Cells, 20:561-572.). For decades, scientists have attempted to grow stem cells in culture to increase the number of cells for transplantation. The challenge of this undertaking lies in the stem cell's predisposition to differentiate. This problem may be associated with the inherent asymmetric cell kinetics of stem cells in postnatal somatic tissues (Sherley, J. (2002) Stem Cells, 20:561-572.). Existing scientific methods used for increasing the number of stem cells include culturing cells on 2-D stromal layers and growing them in the presence of various cytokine cocktails (Rebel, VI, et al. (1994) Blood, 83(1):128-136). However, none of the existing *ex vivo* methods can prevent differentiation of stem cells while promoting proliferation (Rebel, VI. et al. (1996) J Hematother, 5(1):25-37). Methods of screening for compounds useful in culturing stern cells are known. For example , WO 03/104442 discloses a method of screening for compounds using particular tyrasine kinase receptors as a target for selecting binding agents. There is therefore a need in the art for further compounds and methods for identifying compounds capable of propagating and expanding adult stem cells in culture.

### BRIEF SUMMARY OF THE INVENTION

The present invention relates to a method of identifying an agent as capable of preventing differentiation while promoting proliferation of a mammalian stem cell during *ex vivo* culture. The method comprises contacting an agent with at least one of subunits PR72/130 of the serine/threonine protein phosphatase PP2A and detecting binding of said agent to said at least one subunit.

The binding may detected by immunoblotting. Furthermore, the agent may selected from the group consisting of compounds of formula 1-5, 7-12, 15 and 17-28.

### Brief Description of the Drawings

The following drawings do not directly represent embodiments of the invention, but demonstrate the activity of compounds that may be identified according to the methods of the invention and indicate assays that may be used to demonstrate the activity of such compounds.

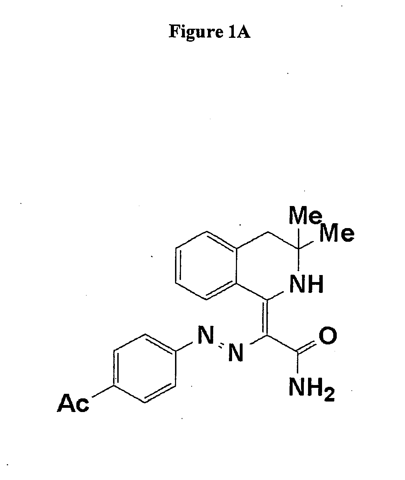

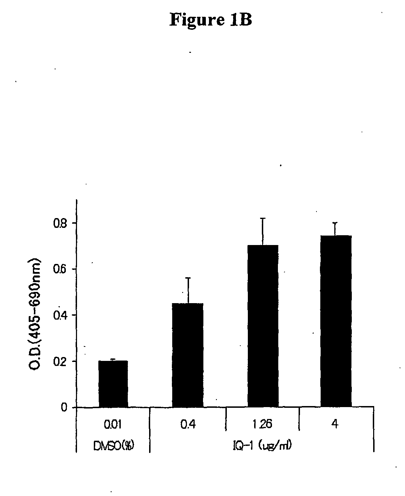

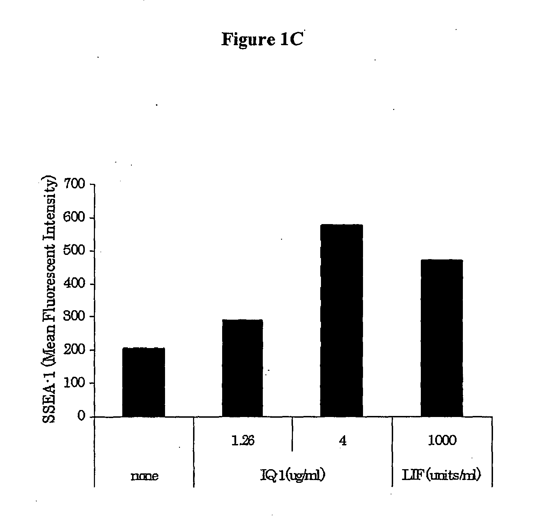

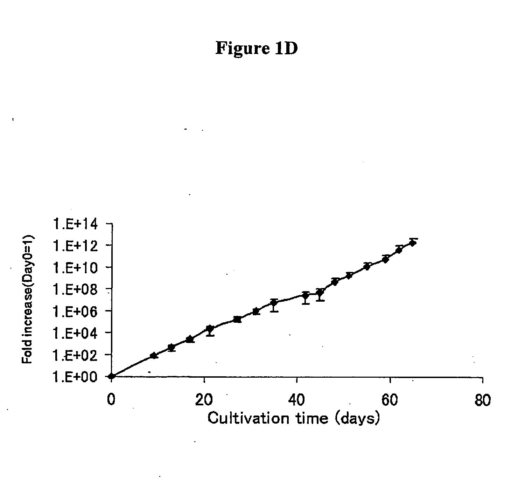

Figure 1. Figure 1A-D shows that IQ-1 maintains undifferentiated state of ESCs. Figure 1A, Structure of IQ-1. Figure 1B, IQ-I dose dependently maintains alkaline phosphatase activity. Figure 1C, IQ-1, dose dependently, maintains SSEA-1 expression. SSEA-1 expression was analyzed 7 days after addition of IQ-1. Figure 1D, IQ-1 enabled ESCs to proliferate in the undifferentiated state for at least 65 days, without MEF feeders or LIF. ESCs, in media supplemented with 4µg/ml IQ-1, were passaged 2-3 times every week at 1×10⁵ -1×10⁶ cells per 6cm dish and counted. All error bars represent mean ±SD.

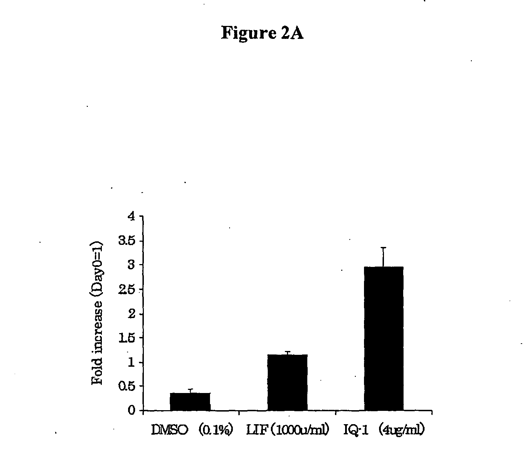

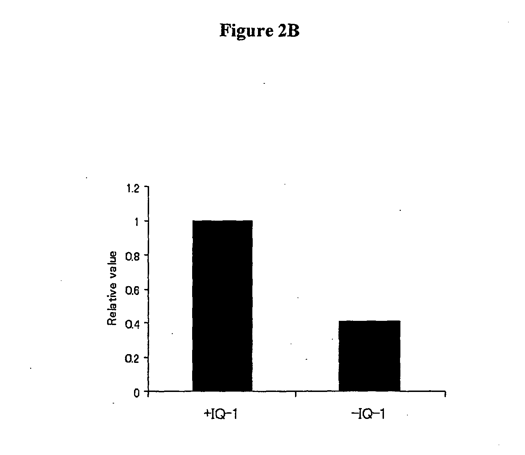

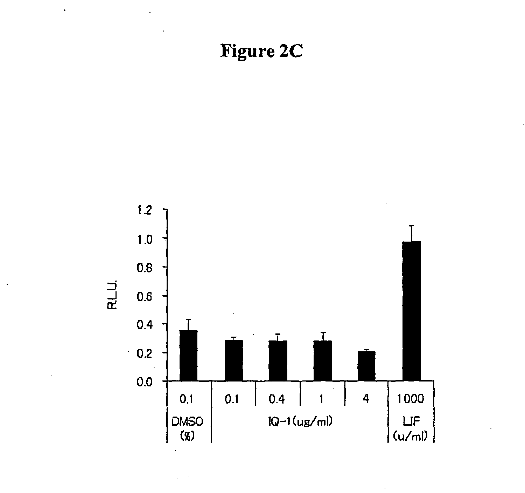

Figure 2. Figure 2A-C shows that IQ-1 maintained ESCs self-renewal independently of LIF. Figure 2A, IQ-1 increases *Nanog* gene expression significantly compared to LIF. mRNA was isolated from ESCs, cultured under feeder-free system, and in the presence of either IQ-1 (4 µg/ml) or LIF (1000 U/ml) for 21 hrs. Real-time RT-PCR for *Nanog* was performed. The control expression level of Nanog at day 0 was set at 1. Figure 2B, removal of IQ-1 decreases *Nanog* gene expression in ESCs. IQ-1 was removed from ESCs which had been cultured under feeder-free system and in the presence of IQ-1 (4 µg/ml), for three days. At the end of this period, mRNA was isolated and real-time RT-PCR was performed to assay for *Nanog* gene expression. Figure 2C, effects of IQ-1 were not mediated through Stat3 signaling pathway as judged by luciferase reporter assay. Feeder-free ESCs, transfected with the pSTAT3-TA-Luc reporter, were exposed to IQ-1 at the indicated doses, or LIF. All error bars represent mean ±SD.

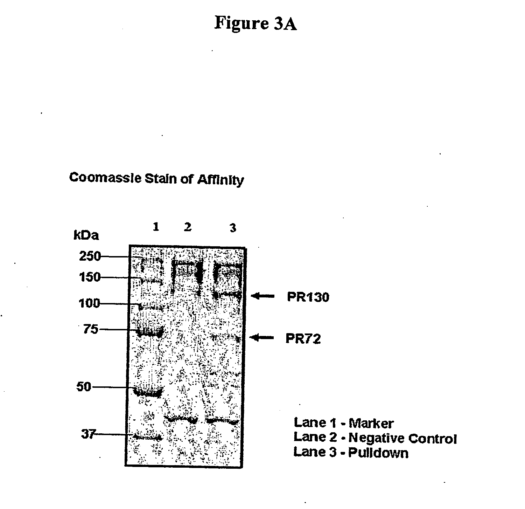

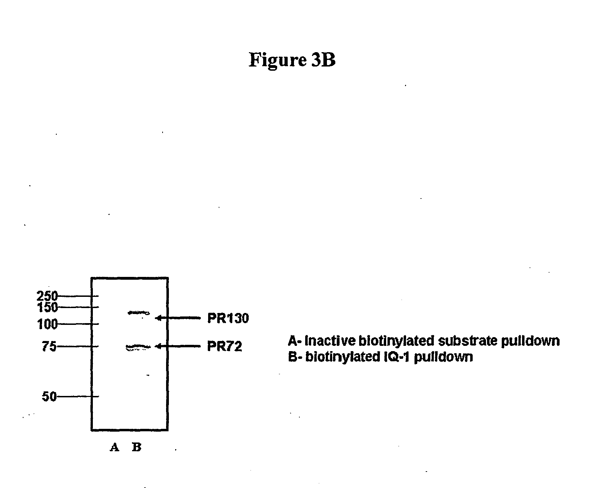

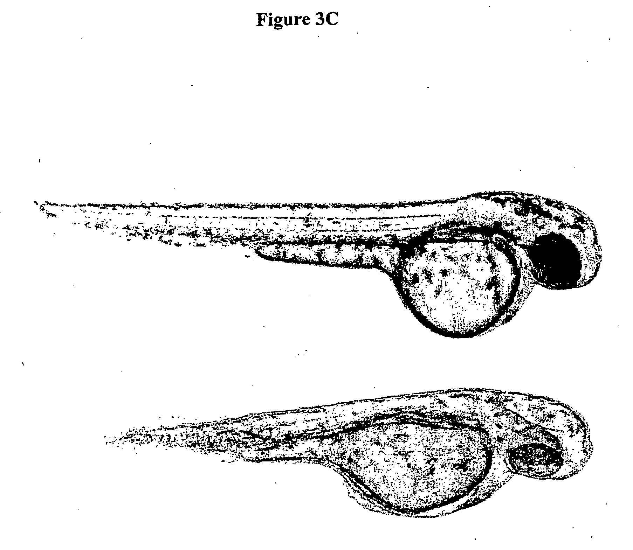

Figure 3. Figure 3A-C shows that IQ-1 modulates Wnt signaling via interaction with PR72/130. Figure 3A, affinity chromatography isolation of IQ-1's molecular target(s) were performed as described in Experimental Procedures. The two bands at 72 kDa and 130 kDa (labeled) were identified by mass spectral sequencing sequencing as the differentially spliced regulatory subunits PR72/130 of the serine/threonine protein phosphatase, PP2A. Figure 3B, immunoblotting, using PR72/130 antisera, was performed to confirm the identity of the two bands. Figure 3C, IQ-1 causes developmental defects in zebrafish. 1-cell stage zebrafish embryos were treated with 1 µM IQ-1 (Lower) or DMSO control (Upper) for 24 hrs. Results are representative of at least 10 embryos, from three independent experiments.

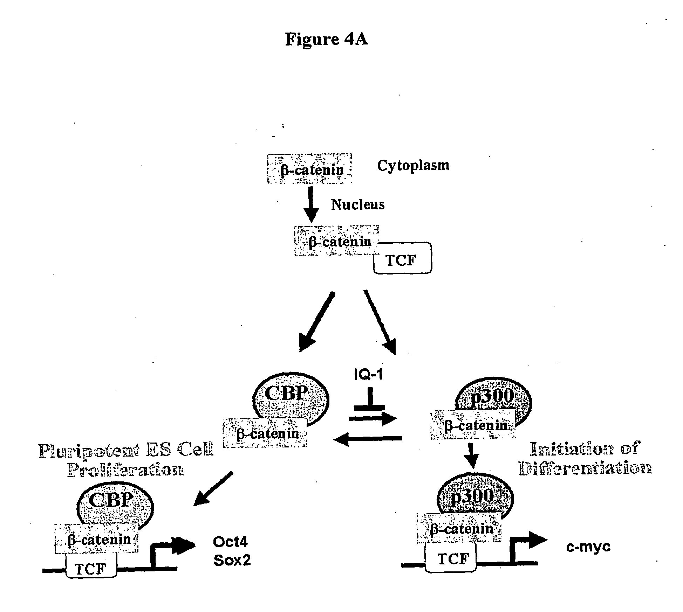

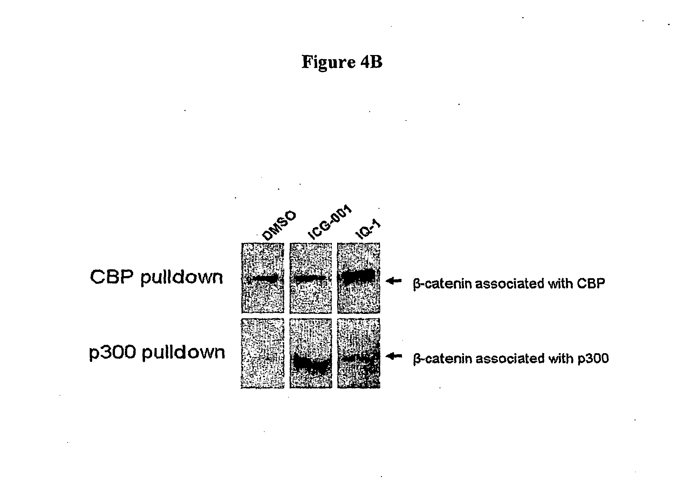

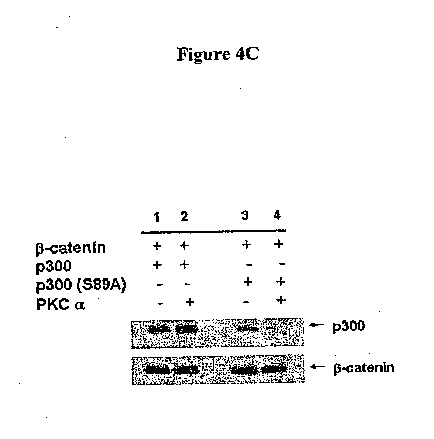

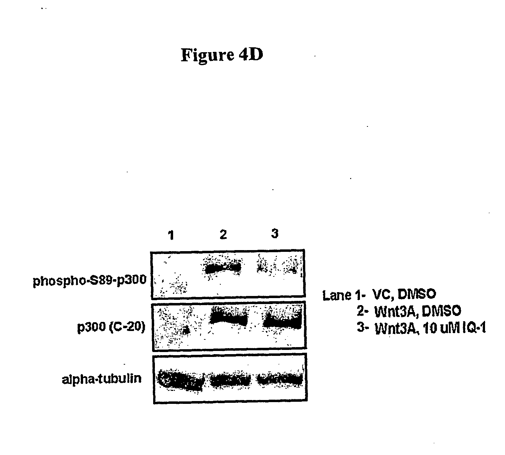

Figure 4. Figure 4A-D shows that IQ-1 maintenance of ESCs is Wnt/β-catenin/CBP dependent. Figure 4A, Wnt/β-catenin Coactivator Switching Model. Figure 4B, IQ-1 increased the CBP/β-catenin complex at the expense of the p300/β-catenin complex. P 19 cells were treated with Wnt3A supplemented with IQ-1, the CBP/β-catenin ICG-001 or DMSO control. Nuclear lysates were co-immunoprecipitated with anti-CBP or anti-p300 antibody and immunoblotted for β-catenin. Figure 4C, phosphorylation of p300 Ser89, in a PKC dependent manner, increased the p300/β-catenin interaction. After in vitro phosphorylation with PKCα, wild type p300 (1-110 aa) and the mutant p300 (p300 S89A) were mixed with P19 lysates and co-immunoprecipitated using the HA-tag. Western blot analysis for p300 (*Upper*) or β-catenin loading control (*Lower*) were performed. Lane 1, p300/ β-catenin binding, Lane 2, PKCα phosphorylated p300/ β-catenin binding, Lane 3, S89A p300/ β-catenin binding, Lane 4, PKCα phosphorylated S89A p300/ β-catenin binding. Figure 4D, IQ-1 decreased the phosphorylation of p300. P19 cells were treated with IQ-1 or DMSO (control) and exposed to purified Wnt3A for 24hrs. Cell lysates were immunoblotted using antibodies specific for p300, or p300 phosphorylated at position Ser 89. Lane 1 negative control, Lane 2, Wnt3a plus DMSO control, Top panel phospho Ser89 p300 immunoblot, Middle panel p300 immunoblot, Lane 3, Wnt3a plus IQ-1, Top panel phospho Ser89 p300 immunoblot, Middle panel p300 immunoblot.

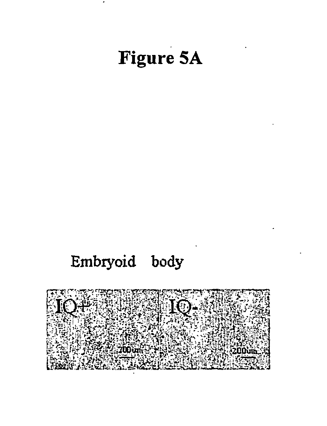

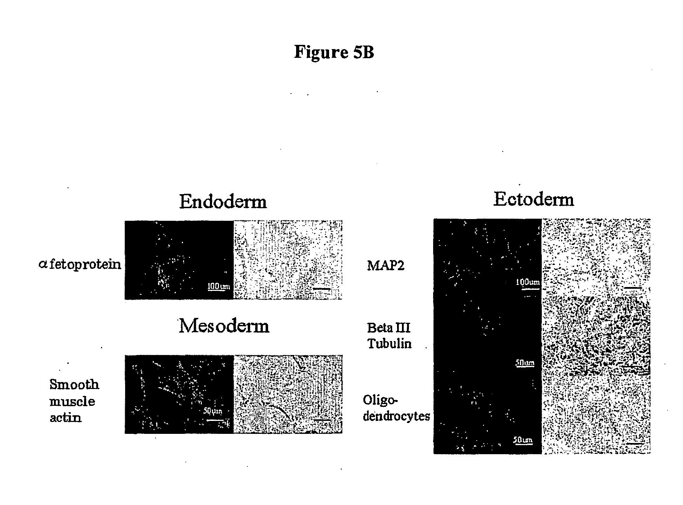

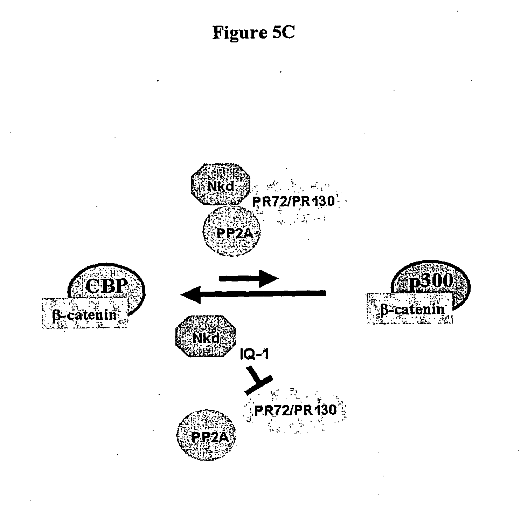

Figure 5. Figure 5A-C shows the pluripotency of long term cultured ESCs. Figure 5A, long term cultured ESCs were induced to form embryoid bodies in suspension cultures for 3 days. ESCs cultured in media in the presence of Wnt3A and IQ-1 (4µg/ml) for 48 days were able to form embryoid bodies (*Left*)*.* ESCs lose their ability to form embryoid bodies after 3 days of culturing in the absence IQ-1 (*Right*). Figure 5B, IQ-1 treated ESCs derived embryoid bodies were cultured (adherence culture) for 7-14 days to induce further differentiation. Immunofluorescence staining for a-fetoprotein, smooth muscle actin, GATA4, MAP2, β-III tubulin and oligodendrocytes demonstrated that long term culture of ESCs in the presence of IQ-1, preserves pluripotency. Figure 5C, model depicting the proposed mechanism of action of IQ-1.

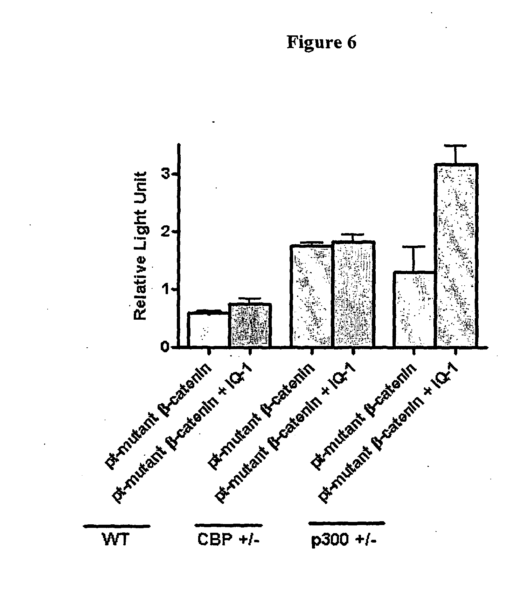

Figure 6. Figure 6 shows TCF/β-catenin reporter gene analysis. The TCF/β-catenin reporter construct TopFlash was cotransfected with a constitutively active β-catenin into NIH-3T3 cells - wt, CBP (+/-) and p300 (+/-) - in the presence or absence of IQ-1. There was no effect of IQ-1 on the Fopflash reporter.

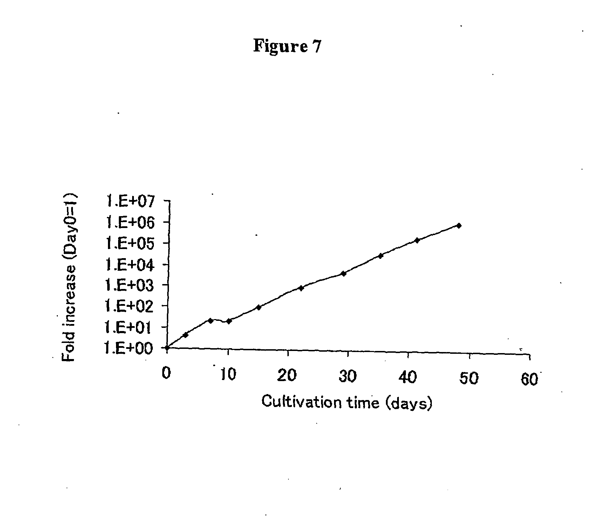

Figure 7. Figure 7 shows long term culture of ESCs in feeder free system with serum-free media supplemented with IQ-1 and purified Wnt3a. ESCs were cultured in 15% KSR, 4µg/ml of IQ-1 and 100ng/ml Wnt3a. ESCs were passaged 2-3 times per week at 1×10⁵ -1×10⁶ cells per 6cm dish and cells were counted.

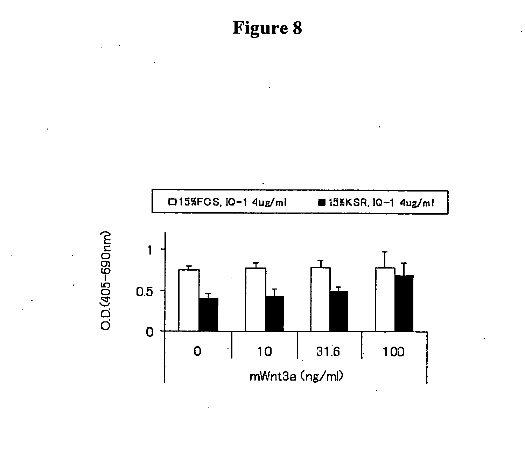

Figure 8. Figure 8 shows that IQ-1 maintenance of ESC proliferation and pluripotency was dependent on Wnt signaling in 15% KSR media. AP activity of ESCs cultured for 7days in IQ-1 containing media supplemented with 15% FCS or 15% KSR. Addition of Wnt3a increases AP activity of ESCs cultured for 7days in 15% KSR containing media to the same level as 15% FCS containing media. (Error bars represent ± S.D.)

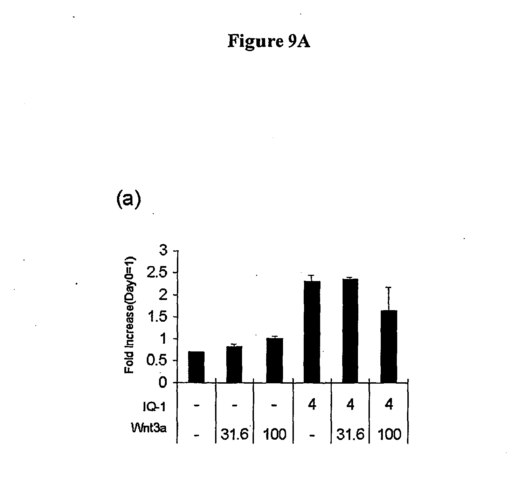

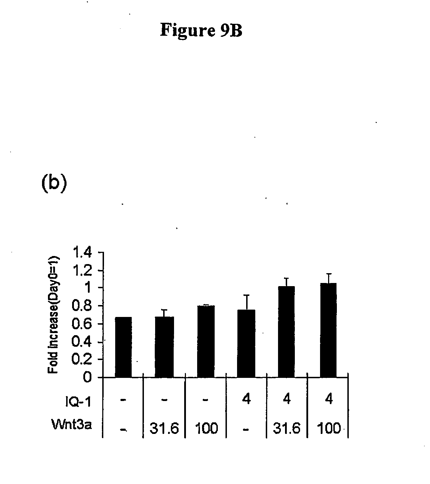

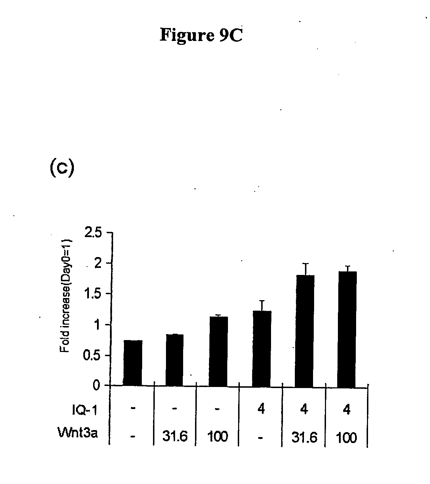

Figure 9. Figure 9 shows real time RT-PCR performed on ESCs cultured in 15% KSR supplemented with IQ-1 and Wnt3a for 42 days without MEF feeders. Over the 42-day culture period, ESCs cultured in the media in the presence of IQ-1 and Wnt3a maintain expression of the pluripotency markers, (a) *Nanog,* (b) *Oct3*/*4* and (c) *Rex-1.* The expression level on day 0 was set as 1.

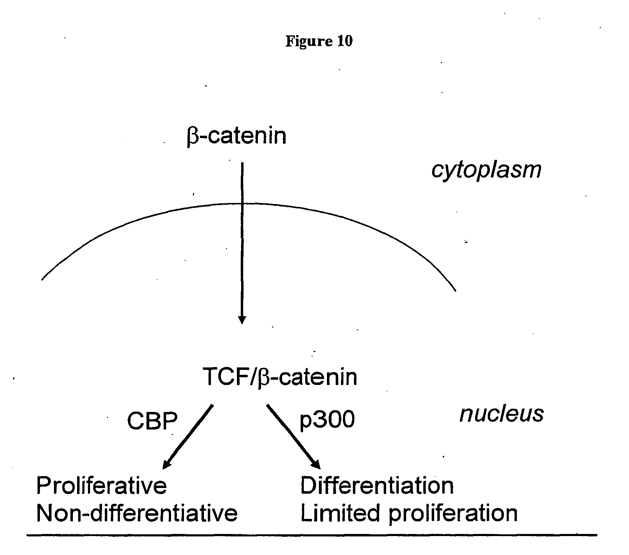

Figure 10. Figure 10 is a schematic representation of two possible mechanisms of action of β-catenin, resulting from alternative interaction with CBP or p300 in the nucleus.

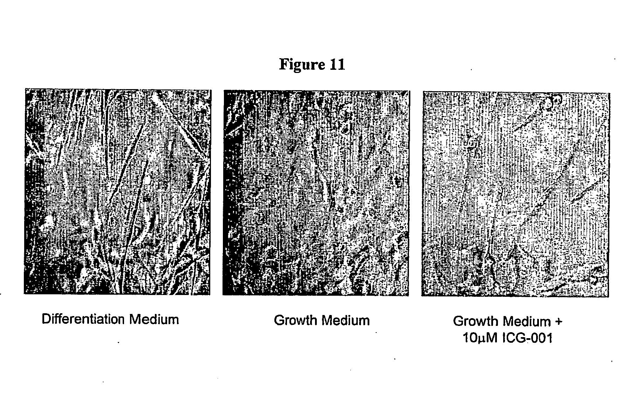

Figure 11. Figure 11 illustrates that ICG-001 at 10µM concentration induced the differentiation of C2C 12 myoblasts in growth medium, similar to differentiation media, and compared to growth medium alone.

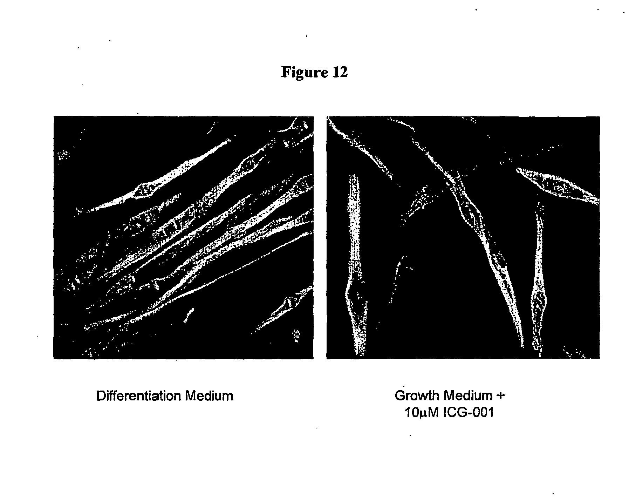

Figure 12. Figure 12 illustrates that differentiation of C2C12 myoblasts was induced by 10µM ICG-001 in growth medium, similar to differentiation medium.

### Detailed Description of the Invention

Stem cells are responsible for the regeneration and maintenance of tissues by balancing the processes of self-renewal (i.e., making new stem cells) and differentiation (i.e., generating cells committed to terminal differentiation). This balance results from integration of regulatory signals intrinsic to the stem cell, as well as extrinsic signals from the microenvironment. Perturbations in the balance between self-renewal and differentiation may result in disease, either as a result of stem cell depletion (e.g., aplastic anemia) or increased self-renewal (e.g., cancer). In neurogenesis, the transcription factors Sox1, Sox 2 and Sox3 play a role in maintaining neural cells in an undifferentiated state.

Most knowledge about the molecular mechanisms of stem cell regulation in mammals has been derived from studies of the hematopoietic system. There is an extensive and expanding understanding of the molecular mechanisms that regulate differentiation along the terminal lineages. However, a mechanistic understanding of the mechanisms that regulate hematopoietic stem cell (HSC) fate decisions is less well understood. A few genes have been identified that, when deleted, result in perturbation of HSC self-renewal (e.g. TNFα-p55-Receptor, p21, Rae28, and Bmi-1) or altered differentiation (e.g., TEL, PU.1, Flt-3, and p27). HoxB4, β-catenin, and Notch signaling, on the other hand, stimulate HSC self-renewal when over-expressed in HSCs.

Recent work has demonstrated that CBP and p300 play important roles in HSC self-renewal and differentiation. CBP and p300 function as molecular integrators of various transcriptional signals. When recruited to promoters by transcription factors, they function as co-activators of transcription through multiple mechanisms, including chromatin remodeling, acetylation of associated proteins, and recruitment of the basal transcription machinery. CBP and p300 are highly homologous on a structural level, with up to 93% identity within certain protein-binding domains (SEQ ID NO: 1 and 2). For most functions, the two proteins appear to be functionally redundant. However, mouse genetic loss-of-function studies demonstrated a difference between p300 and CBP function in HSCs: loss of CBP results in defective HSC self-renewal, whereas loss of p300 results in defective hematopoietic differentiation.

CBP and p300 have been previously shown to interact with many of the known transcription factors shown to be important in HSC regulation (e.g., HoxB4, β-catenin, Notch, AML-1, MLL). Earlier results suggest that within HSCs there may be transcription factors that are specifically co-activated by CBP that are critical for self-renewal, and others that are preferentially co-activated by p300 that are critically required for differentiation. One example of a signaling pathway that seems to utilize CBP and p300 differentially is the Wnt signaling pathway. The Wnt signaling pathway has been shown to play a pertinent role in the development and maintenance of various tissues, including blood, intestines, and skin. Its effects are executed at the level of stem and progenitor cells, affecting both self-renewal and differentiation. Moreover, the importance of Wnt signaling in maintaining the undifferentiated features of embryonic stem (ES) cells has been well established. Importantly, when Wnt signaling is perturbed it can lead to the development of cancer in these same tissues.

β-catenin (SEQ ID NO:3) is a vertebrate homolog of *Drosophila* gene armadillo, which functions in both cell adhesion and, as discussed herein, the Wnt signaling pathway. γ-catenin (SEQ ID NO:4) is also a vertebrate homolog of armadillo. β-catenin and γ-catenin have analogous structures and functions, and they have the ability to be regulated by the APC tumor suppressor.

Activation of the Wnt signaling pathway requires the nuclear stabilization of TCF (T cell factor)/β-catenin complexes and recruitment of transcriptional co-activators, such as CBP and p300. β-catenin is constitutively produced in the cell, and inhibitory mechanisms exist to maintain β-catenin levels at below those that would lead to aberrant transcriptional activity in vivo, leading to pathological conditions such as cancer. In one example of aberrant regulation, Emami and colleagues (PNAS, 101,12682-7, 2004) recently demonstrated that β-catenin preferentially associates with CBP in cancer cells. However, when β-catenin was prevented from associating with CBP, by utilizing a β-catenin/CBP-specific inhibitor, β-catenin could bind to p300. The "alternative" binding of β-catenin to p300 was accompanied by the execution of a differentiative genetic program (Teo et al. PNAS submitted). Thus, β-catenin is thought to promote proliferation without differentiation by binding to and activating CBP, and to initiate differentiation with limited proliferation by binding to and activating p300. Perturbation of β-catenin interation with CBP and/or p300 is expected therefore to influence differentiation or proliferation.

Stem cell therapy is based on the ability of human fetal or adult pluripotent stem cells to differentiate into a variety of cell types. Stem cells may be used to replace damaged cells as a treatment for many different diseases including cancer, Parkinson's disease, spinal cord injury, bums, diabetes, heart disease, rheumatoid arthritis, and osteoarthritis and for gene therapy (Lazic, S.E. et al. J Hematother Stem Cell Res, 12(6):635-642, Gafni, Y. et al. Gene Ther, 11(4):417-426). Stem cell therapy has long been an exciting potential medical breakthrough. The ability to inject normal stem cells into a patient, where they could generate organ-specific cells to potentially replace defective patient tissues, offers enormous potential.

Embryonic stem cells (ES cells) represent an important research tool and a potential resource for regenerative medicine. Generally, ES cells are cocultured with a supportive feeder cell layer of murine embryonic fibroblasts (MEFs). The MEF feeder layer supplies factors which maintain the ES cell's capacity for self renewal and block spontaneous differentiation. These cumbersome conditions, as well as the risk of xenobiotic contamination of human ES cells grown on MEFs, make it a priority to develop chemically defined media that can be safely utilized for the expansion of ES cells. Using a high throughput cell based screen, the small molecule IQ-1 was identified as a compound that allowed for the expansion of mouse ES cells without a MEF feeder layer or the addition of leukemia inhibitory factor (LIF), and prevented spontaneous differentiation. It has also been determined that IQ-1 prevents β-catenin from switching coactivator usage from CBP to p300. The increase in β-catenin/CBP mediated transcription at the expense of β-catenin/p300 mediated transcription is critical for the maintenance of stem cell pluripotency.

ES cells derived from the inner cell mass of embryos can be cocultured on embryonic fibroblast cell layers. More recently, Xu et al. (Xu et al. Nat. Biotech. 2001, 19, 971) demonstrated that human ES cells can be grown under feeder free conditions utilizing MEF conditioned media. However variations in MEFs and MEF conditioned media, the lack of knowledge about what factors in conditioned media are important and concerns about zoonotic contamination emphasize the need for chemically defined conditions to expand ES cells.

The ability to maintain adult skin stem cells *in vitro* has allowed engraftment of cultured skin onto bum victims (Green, H. (1991) Sci Am, 265:96-102). Additionally, at present, there are three adult stem cell related transplantation procedures used for hematopoietic reconstititution: bone marrow transplantation (BMT), peripheral blood stem cell transplantation (PBSCT) and umbilical cord blood stem cell transplantation (UCBSCT). The first two hematopoietic reconstitution techniques, BMT and PBSCT, suffer from a significant matching problem with allogeneic donors. The degree of match required for a successful transplant appears to be less stringent for UCBSCT than BMT or PBSCT. However, the relatively lower volume of harvested stem cells and the availability of only one collected cord blood unit per transplant procedure limit the wide applicability of UCBSCT (McCaffrey, P. *Lancet Oncol*., 6 (1) : 5, 2005). One solution to this problem is *ex vivo* expansion of the cord blood stem cells. However, there is a significant hurdle to overcome in order to provide this straightforward solution.

### Stem Cells and Cancer "Stem Cells"

A unifying feature of all cancers is their capacity for unlimited self-renewal, which is also a defining characteristic of normal stem cells. Decades ago, it was discovered that the proliferative capacity of all cancer cells was not equivalent, and only a small minority of tumor cells were able to proliferate extensively (Hamburger) AW, et al. (1977) Science, 197(4302):461-463). This gave rise to the concept that malignant tumors are comprised of *Cancer Stem Cells*, which have great proliferative potential, as well as another pool of more differentiated cancer cells, with limited proliferative capacity. An important implication of the *Cancer Stem Cell* hypothesis is that there are mechanistic similarities between the self-renewal of normal stem cells and the proliferation of cancer stem cells (Pardal, R. et al. (2003) Nat Rev Cancer, 3(12):895-902). Recent studies have demonstrated that specific gene products regulate both the self-renewal of normal somatic stem cells, as well as the proliferation of cancer cells (Park, IK. et al. (2003) Nature 423:302-305; Lessard, J. et al. (2003) Nature, 423(6937):255-260). This implies that similar mechanisms are utilized in both stem cells and cancer cells to maintain a proliferative, non-differentiated state.

### Wnt Signaling in Stem Cells and Cancer

The Wnt/β-catenin pathway initiates a signaling cascade critical in both normal development and the initiation and progression of cancer (Giles, RH et al. (2003) Biochim Biophys Acta, 1653(1):1-24; Wodarz, A. et al. (1998) Annu Rev Cell Dev Biol, 14:59-88). Wnt signaling and in particular the nuclear functions of β-catenin have been shown to be important in the maintenance, proliferation as well as the differentiation of stem cells (Song, X. et al. (2003) Development, 130(14):3259-3268). Some of the salient features of this signaling pathway, are summarized in Figure 1. The Wnt/β-catenin pathway normally regulates expression of a range of genes involved in promoting both proliferation and differentiation. Activation of the Wnt pathway allows β-catenin to accumulate in the nucleus, bind to members of the TCF family of transcription factors, and form a transcriptionally active complex, by recruiting either the transcriptional coactivator CBP or its closely related homolog, p300. However, in greater than 85% of colon cancers, mutations in this pathway lead to constitutive activation and expression of target genes, e.g. c-myc, cyclin D1 and survivin, all of which are critical for rapid cell proliferation (Kolligs, FT. et al. (1999) Mol Cell Biol, 19(8):5696-5706; Tetsu, O. et al. (1999) Nature, 398(6726):422-426; Kim, PJ. et al. (2003) Lancet, 362:205-209). Thus, tumorigenesis in the intestinal epithelium appears to be caused by Wnt/β-catenin induced hyper-proliferation of intestinal crypt stem cells, followed by accumulation of additional mutations that confer malignancy and cancer progression. Wnt signaling has also been demonstrated to be important for the maintenance of pluripotency in both mouse and human embryonic stem cells in culture (Sato, N. et al. (2004) Nat Med, 10(1):55-63). Expression of multiple components of the Wnt pathway is evident in the P19 human embryonal carcinoma cell lines, as well as in embryonic stem cells (Walsh, J. et al. (2003) APMIS, 111(1):197-211).

### Wnt and Hematopoietic Stem Cells (HSC)

The self-renewal of hematopoietic stem cells (HSC) is also promoted by Wnt signaling. Overexpression of stabilized β-catenin in cultured bone marrow HSC from mice increased the number of these cells in long-term culture as measured by their ability to reconstitute the hematopoietic systems of mice following irradiation. Additionally, purified Wnt3a promoted self-renewal but only partially inhibited the differentiation of HSC in culture (Reya, T. et al. (2003) Nature, 423(6938):409-414).

### Differential Coactivator Usage in Wnt/β-catenin Signaling

As discussed above, the functions of CBP and p300 have been described as redundant in several studies (reviewed in Goodman, RH. et al. (2000) Genes Dev, 14(13):1553-1577) and their expression pattern during mouse development is almost identical (Partanen, A. et al. (1999) Int J Dev Biol, 43(6):487-494). However, it is becoming increasingly clear that these highly homologous coactivators are not redundant under physiological conditions, and are responsible for distinct transcriptional programs: Rebel *et al*. (Rebel, V.I. et al. (2003) Proc Natl Acad Sci U S A, 99(23):14789-14794), using cells from knockout mice, demonstrated that a full dose of CBP, but not p300, is crucial for HSC self-renewal. Conversely, p300 but not CBP, is essential for proper hematopoietic differentiation. Similarly, Eckner and colleagues (Roth, J.F. et al. (2003) Embo J, 22(19):5186-5196) demonstrated a critical role for p300's histone acetyltransferase activity (HAT) but not CBP's HAT activities. These studies and others clearly demonstrate that CBP and p300 play non-redundant and distinct roles during development.

From the inventor's previous chemogenomic studies with the small molecule inhibitor of the β-catenin/CBP interaction and additional gene expression profiling, a model was developed that describes how differential coactivator usage in Wnt signaling controls proliferation vs. differentiation. The critical feature of this model is that the CBP arm (Figure 10, left side) of the pathway is essential for proliferation without differentiation, for example in cancer or stem cells, whereas the p300 arm (Figure 10, right side) is critical for differentiation, with limited proliferation. ICG-001 specifically inhibits β-catenin/CBP dependent transcription (i.e. the left arm of the pathway), thus selectively inducing programmed cell death in cancer cells (Emami, K.H. et al. (2003) Proc Natl Acad Sci USA, 101, 12682-7, 2004), and inducing the differentiation of non-tumorigenic precursor cells, e.g. C2C12 myoblasts (Figs. 11 and 12) and 3T3-L1 preadipocytes.

Without being bound by a specific mechanism, the premise is that selectively inhibiting or down-modulating the β-catenin/p300 interaction (i.e. the right side of the pathway, Fig. 10) allows for proliferation without differentiation of pluripotent stem cells. The invention is also in part on the discovery that one mechanism involves the selective binding of a compound or agent to one or more of the PR72/130 subunits of the serine/threonine protein phosphatase PP2A, as disclosed in more detail in Example 1 herein. The agent may have activity similar to IQ-1.

The serine/threonine phosphatase PP2A is involved in regulating intracellular signaling, gene expression and cell cycle progression. A major function of PP2A is to regulate signaling cascades by opposing the activity of serine/threonine kinases (20). PP2A consists of a multisubunit complex. The core components of this trimeric complex are a 36 kDa catalytic, a 65 kDa regulatory (PR65) and a third variable subunit, one of which is PR72/130. PR72/130 represents tissue selective differentially spliced forms of the same gene (21). PP2A regulates the Wnt signaling cascade at multiple levels (22, 23). Recently, PR72/130 has been shown to interact with the protein Naked cuticle (Nkd), a negative regulatory component of the Wnt signaling pathway (24), thereby modulating Wnt signaling (25). Creyghton et al., using morpholinos in xenopus embryos, demonstrated that PR72 like Nkd, is a "negative" regulator of "canonical" Wnt/β-catenin signaling and is involved in the switch from "canonical" Wnt signaling to "non-canonical" convergent extension (25).

For maintenance of hematopoietic stem cell proliferation, an agent may "modulate" the proliferation of stem cells by affecting the post-translational modifications of any one of CBP, p300, or β-catenin, leading to a selective increase of β-catenin interaction with CBP or a selective decrease of β-catenin interaction with p3 00, wherein the agent does not directly bind to CBP or p300. By "interact" and "interaction" is meant the normal biological relationship between two or more molecules, in this case β-catenin with CBP or p300. The agent may increase the binding of β-catenin to CBP, or the agent may decrease the binding of β-catenin to p300. Either way, the overall result biases the β-catenin pathway towards "proliferative/non-differentiative program" of the target cells, which may be adult stem cells, such as hematopoietic stem cells, neural stem cells, or skin stem cells. An agent will "modulate" the proliferation of stem cells if the stem cells undergo more proliferation and/or less differentiation that in the absence of the agent. For example, with reference to Figure 5C, preferential binding of β-catenin to CBP, with less binding to p300, is associated with maintaining hematopoietic stem cells in an undifferentiated state wherein they undergo continuous proliferation, resulting in enhanced numbers of undifferentiated cells useful for repopulating the hematopoietic system of a mammal, such as a human, in need of such treatment. Such modulation can be measured using, for example, assays described in the Examples herein.

Agents suitable for such use can be screened using co-immunoprecipitation methods as described in Emami et al. PNAS, 101, 12682-7, 2004. Briefly, target cells, in this case HSC, are transfected with full-length β-catenin or with full-length p300. Nuclear lysates are treated with a radiolabeled test agent alone, or with cold test agent. Unbound radiolabeled test agent is removed, and incorporation of the radiolabeled test agent is measured. The results indicate whether the test agent specifically interacts with p300.

A separate series of experiments can demonstrate inhibition of the interaction of β-catenin with p300. The minimal binding domain of CBP (amino acids 1-111), p300 (amino acids 1-111) and the C-terminal region of β-catenin (SEQ ID NO:3) (amino acids 647-781) are expressed in mammalian cells treated with the appropriate agents to modify the interaction and purified. β-catenin is bound to protein A-agarose beads coated with β-catenin-specific antibody and incubated with either CBP or p300. Unbound proteins are removed by washing, then the specific interactions between β-catenin and p300, and β-catenin and CBP, are challenged using the test agent, for testing the Compounds which directly bind to CBP or phosphor Ser89 p300. Agents that either increase the binding of β-catenin to CBP or decrease the binding of β-catenin to p300 are further tested in vitro using a suitable model of hematopoietic stem cell proliferation/differentiation. One such model is described in Rebel, V. I. et al., PNAS 99:14789-14794, 2002.

Suitable agents may achieve the desired biological effects through one of several mechanisms. In each case, reference to "increase" or "decrease" refers to the assay results or biological effects relative to the values in the absence of the agent. For example, the agent may increase the binding of β-catenin to the ammo-terminal 110 amino acids of CBP, or it may decrease the binding of β-catenin to the amino-terminal 110 amino acids of p300. The decrease in binding of β-catenin to p300 may be achieved by inhibiting the phosphorylation of Ser 89 of p300, wherein the phosphorylation is catalyzed by protein kinase C-epsilon (PKC), calcium/calmodulin-dependent protein kinase (CaMK), protease-activated receptor-4 (PAR-4), protease-activated receptor-1 (PAR-1), or other serine/threonine protein kinase either directly or indirectly via a kinase cascade.

The decreased phosphorylation of Ser 89 of p300 may be achieved by increasing the phosphorylation of Ser 90, for example by mitogen-activated protein kinase 4 (MAPK), cyclin-dependent kinase (CDK), or other serine/threonine protein kinase.

A preferable agent may modulate the interaction of Ser 89-phosphorylated p300 with 14-3-3 proteins. Such agents may be analogs of Fusicoccin. Fusicoccin is a fungal toxin that is used to study H+-ATPase activation. The mechanism involves inducing an irreversibie bond between the C-terminal portion of H+- ATPase, and 14-3-3 protein. (Svennilid, F. et al., Plant Cell 11:2379-2392, 1999.) As a result, the C-tenninal auto-inhibiting domain is displaced. Similarly, analogs of Fusicoccin may modulate the interaction of Ser-89 phosphorylated p300 with 14-3-3 proteins, resulting in the decreased interaction of p300 with β-catenin.

The agent may modulate the interaction of Pin 1 with β-catenin or with CBP or p300. For example, the agent may increase the association of Pin1 with β-Catenin/CBP. Pinl (prolyl isomerase) has been implicated in cancer mechanisms by inhibiting the interaction of β-catenin with the tumor suppressor APC. Pin1 overexpression has been reported to occur in human breast cancer. (Ryo, A. et al., Nat. Cell Biol. 3:793-801 (2001)). Pinl has also been implicated in normal spermatogenesis. Atchison, F.W. et al. (Biol. Reprod. 69:1989-1997, 2003) reported that adult Pinl-deficient mice exhibited evidence of accelerated exhaustion of stem cell potential, and possible bias towards the differentiation pathway in the absence of Pin1.

Phosphorylation affects the conformation of proteins and creates conditions for binding of signal transducers to certain suitable domains capable of recognizing the phosphorylated residue or residues. Pin1 specifically recognizes phosphorylated S/T-P bonds (Ser/Thr-Pro motifs), For example, Pinl directly binds a phosphorylated Ser-Pro motif (Ser 246-Pro) next to the APC-binding site in β-catenin, inhibits β-catenin interaction with adenomatous polyposis coli protein (APC), and thereby increases its translocation into the nucleus. (Ryo, A. et al., Nature Cell Biol. 3:793-801, 2001.)

Pin1 can also affect coactivator interactions with transcription factors. P73 is a transcription factor related to the tumor suppressor p53. Pinl-modified p73 displayed a higher affinity for p300 than unmodified p73. (Montovani, F. et al., Mol. Cell 14:625-636, 2004.) Similarly, Pin1 binding to phosphorylated β**-**catenin can increase the β-catenin/CBP interaction and thereby β-catenin/CBP dependent gene transcription promoting proliferation at the expense of differentiation.

Such agents can be incorporated into biomaterials on which hematopoietic stem cells are grown. Examples are disclosed in Horak et al., Biomaterials 25, 5249-60, 2004 and Harrison et al. Biomaterials 25, 4977-86, 2004.

Although hematopoietic stem cells are disclosed herein as a target of the above-mentioned methods, the methods are applicable to any adult mammalian stem cells (or ES cells not derived from human embryos that can be used for tissue regeneration. Adult stem cells constitute an undifferentiated population of cells that retain the ability to proliferate throughout postnatal life and to differentiate into specialized cells to replace cells that become diseased, die or are lost. (Agrawal, S. et al; Trends in Biotechnology 23:78-83, 2005.) In addition to HSC, stem cells suitable for use in the above methods include neural stem cells, skin stem cells, muscle stem cells, and pancreatic islet cells.

The goal of diabetes treatment is to restore normal numbers and function of insulin-producing β cells. Trucco, M. (J. Clin. Invest. 115:5-12, 2005) discusses the existence of adult pancreatic precursor cells that can generate β cells, and are referred to as pancreas-derived multipotent precursors. Other stem cells may be induced to direct their differentiation toward the β cell. For either of these sources of β cells, the methods and agents discussed above are suitable for inducing proliferation and limiting differentiation, in order to achieve a suitable number of cells for therapeutic use.

Adult neural stem cells can differentiate into neurons, astrocytes, and oligodendrocytes, which are the three major lineages of the adult nervous system. It may be appropriate to manipulate adult neural stem cells *in situ* in order to achieve neurogeneration *in vivo*. Active stem cells exist in adult brain in the dentate gyrus region of the hippocampus and the subventricular zone of the forebrain, and these stem cells can differentiate into neurons, astrocytes and oligodendrocytes. In addition, quiescent stem cell pools exist in the spinal cord, substantia nigra, optic nerve, and hypothalamus. (Agrawal, S. et al., 2005). Thus, defined pools of neural stem cells are available for modulation .

Skin stem cells may be induced to proliferate *in vivo* in order to enhance or restore hair growth. Recent evidence suggests that the Wnt pathway is involved in the ability of skin epithelial cells to acquire and/or maintain characteristics of multipotent stem cells. (Alonso, L. et al.; PNAS 100:11830-11835, 2003). Multipotent stem cells in skin receive Wnt signals before they commit to form hair follicles. In transgenic mouse skin in which β-catenin is constitutively stabilized, adult interfollicular epidennis takes on characteristics of embryonic skin, and may have the capacity to develop into hair follicles. (Gat, V., Cell 95:605-614, 1998). Thus, agents and methods discussed above are suitable for enhancing the proliferation of multipotent stem cells in the skin, to provide a reservoir of cells capable of forming hair follicles in order to increase or replace lost h air growth, including in vivo applications, for example by topical use. U.S. Patent No. 6,419,913 discloses compositions suitable for topical delivery of therapeutic agents including agents for treatment of hair loss. U.S. Patent No. 6,680,344 also discloses topical delivery of agents for treating hair loss.

In addition to using stem cells following proliferation induced by the agents and methods discussed above, it is also feasible to alter the stem cells prior to use, by gene therapy. Methods to enhance the proliferation of mammalian stem cells expressing an exogenous gene, prior to administration of the cells for therapeutic use are contemplated. The gene therapy may also be conducted *in vivo*, for example, to alter the differentiation potential of neural stem cells. (Gomes, W.A. et al., Dev. Biol. 255:164-177, 2003; Pardridge, W.M., Curr. Opin. Drug Discov. Devel. 6:683-691, 2003.)

An assay suitable for determining whether mammalian stem cells are maintained in a non-differentiated state involves the use of a reporter gene under the control of the OCT4 promoter. OCT4 is a known marker of the undifferentiated stem/progenitor cell state, and the promoter region can be functionally linked to a reporter gene such as EGFP (enhanced green fluorescent protein) as described in Gerrard, L. et al., Stem Cells 23:124-133 (2005)), or luciferase. Using either reporter gene, cells are transfected with an OCT4-reporter gene construct using methods described in Gerrard et al. (2005) and the effect of suitable agents on the undifferentiated versus differentiation state of the cells can be tested.

Methods for testing the effect of small molecules on stem cells *in vitro* include those described by Chen, J.K. et al., P.N.A.S. 99:14701-14076 (2002) and Franlt-Kamenctsky, M. et al., J. Biol 1:10 (2002).

Embryonic stem cells represent an important tool for research and in principle, a potential resource for regenerative medicine (Hori Y et al Proc. Natl. Acad. Sci. USA 2002, 99, 16105, Kim JH et al. Nature, 2002, 418, 50, Lanza RP et al, Nat Med, 1999, 5, 975). Murine ES (mES) cells, derived from the pluripotent inner cell mass, can be grown in the absence of feeder cells in media supplemented with serum and LIF. The ability of LIF to maintain mES cell pluripotency, requires activation of the STAT3 signaling pathway. However, LIF does not maintain the undifferentiated state of hES cells despite the fact that it activates the STAT3 signaling pathway (Humphrey R and Beattie G Stem Cells 2004, Daheron L and Opitz S Stem Cells 2004).

As shown in detail in the Examples, IQ-1 dose dependently maintained ES cell proliferation and pluripotency, IQ-1 could maintain ES cell proliferation in the absence of serum if the media was supplemented with Wnt3a. Importantly, Wnt3a plus IQ-1 was sufficient to maintain ES cell proliferation and pluripotency for extended periods of time in culture (at least for 48 days). Wnt3a plus IQ-1 increased the expression of Oct4 and Sox2 and decreased the expression of c-myc in P19 embryonic carcinoma cells. Recently, Boyer et al. (Cell 122, 947, 2005) demonstrated that Oct4 and Sox2 co-occupy the promoters/enhancers of a substantial portion of the genes required for the maintenance of human ES cells. Furthermore, c-myc appears to be a critical player in the balance between stem cell self renewal and differentiation and increased upon differentiation (Wilson A et al Genes Dev 18, 2747, 2004).

Canonical or Wnt/β-catenin signaling plays a crucial role in regulating the expansion of both human and mouse stem cell populations (Kleber M and Sommer L Current Op Cell Bio 2004, Willert K and Brown J Nature 2003, and Sato N and Meijer L Nat. Med 2004). However, numerous studies have demonstrated that Wnts can act as either a growth factor maintaining pluripotency or alternatively induce differentiation and influence cell lineage decisions (Kleber and Sommer 2004, Ille F and Sommer L Cell Mol Life Sci 2005, Murashov A, Pak E Faseb J 2004, Feng Z and Srivastava A BBRC 2004).

Recently, the inventors developed a model to explain these divergent Wnt/β-catenin signaling activities (Fig. 5). This model highlights the distinct roles of the coactivators CBP and p300 in the Wnt/β-catenin signaling pathway (Emami et al 2004, Ma et al 2005, Teo et al 2005, McMillan and Kahn 2005). The critical feature of the model is that TCF/β-catenin/CBP mediated transcription is critical for stem cell/progenitor cell proliferation, whereas a switch to TCF/β-catenin/p300 mediated transcription, whether induced chemogenomically with ICG-001 or endogenously, is critical to initiate a differentiative program with a more limited proliferative capacity. Based on this model, experiments were performed to determine whether IQ-1 affected selective CBP coactivator usage in the Wnt/β-catenin signaling pathway.

IQ-1 selectively promoted the β-catenin/CBP interaction at the expense of the corresponding p300/β-catenin interaction. Using an affinity chromatography approach, it is shown herein that the molecular targets of IQ-1 are the differentially spliced regulatory subunits PR72/PR130 of the protein phosphatase PP2A (Bernards R 2004 and in press). Bernards has previously demonstrated that PR72 interacted with the protein Nkd, a Wnt-inducible antagonist of Wnt/β-catenin signaling, and that loss of either PR72 or Nkd resulted in activation of Wnt/β-catenin signaling. IQ-1 had dramatic effects on zebrafish embryonic development and convergent extension. By identifying PR72/130 as molecular targets of IQ-1, the invention provides an assay for identifying other agents useful in promoting and/or maintaining stem cell proliferation.

The ability to expand "stem/progenitor" populations under defined growth conditions has important ramifications in the area of regenerative medicine (Hori Y et al Proc. Natl. Acad. Sci. USA 2002, 99, 16105, Kim JH et al. Nature, 2002, 418, 50, Lanza RP et al, Nat Med, 1999, 5, 975). The Wnt signaling pathway clearly plays an important role in the expansion of "stem/progenitor" populations (Reya T et al. Nature, 2005, 434, 843). However, Wnt signaling is also critical in the differentiation processes and development of cells, tissues and organs. These divergent behaviors of Wnt signaling are apparently controlled via selective usage of the coactivator proteins CBP or p300 (Ma et al., Teo et al. and McMillan and Kahn 2005). The endogenous choice of coactivator usage appears to be controlled by a complex array of differential post-translational modifications.

Pharmacologically, the inventor previously showed direct coactivator selection by blocking the CBP/β-catenin interaction with ICG-001, thereby forcing a switch to the p300/catenin interaction, which has divergent promoter specific effects (Ma et al.). The present results demonstrate that IQ-1, through modulation of post-translational modifications and interaction with components of Wnt/β-catenin inhibitory feedback, can also manipulate Wnt/β-catenin coactivator selection. Enhancing Wnt/β-catenin/CBP signaling and preventing the switch to Wnt/β-catenin/p300 usage by IQ-1 allows for the long term expansion of ES cells while maintaining pluripotency in defined media without MEFs or serum. The controlled proliferation of "stem/progenitor" cells for the production of the raw materials required for regenerative medicine is an important application.

Additional compounds useful for practicing the methods described and claimed herein include compounds disclosed and taught in Japanese patent publication JP2006/180763A2, filed December 27, 2004. Compounds of the patent publication include those designated as compounds with the structures as disclosed in JP2006/180763A2; examples are shown below.

Compounds include the following, designated below as formula (compound) numbers. 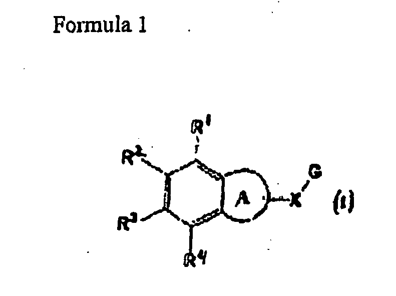 where R¹, R², R³ and R⁴ may be the same or different; and represent an electron attractive group, an electron donating group or a hydrogen atom. Ring A indicates a 5 to 8 member ring containing inside the ring at least one hetero atom. X indicates an alkylene group with 0 to 10 atoms on the main chain. An alkylene group with 0 atoms indicates a single bond. Ethylene configured of at least one the said alkylene groups may be substituted by - C = C- group and / or - N = N - group and / or - CONH - group. In addition, it may be a double bond group which bonds with ring A. In addition, the alkylene group may have at least one electron attractive group, electron donating group or hydrogen atom as a substitution group. G is an aromatic group which may have an electron attractive group, electron donating group or hydrogen group. Said ring A may have at least one electron attractive group and / or electron donating group as a substitution group instead of a-XG group. 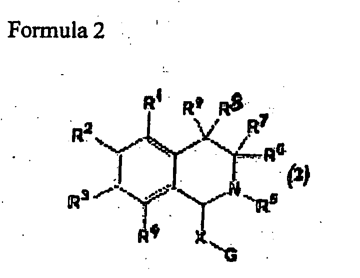 where R¹, R², R³ and R⁴, X and G are the same as defined in Compound 1 above. R⁵, R⁶, R⁷, R⁸ and R⁹ may be the same or they may be different and they indicate an electron attractive group, an electron donating group or a hydrogen atom. 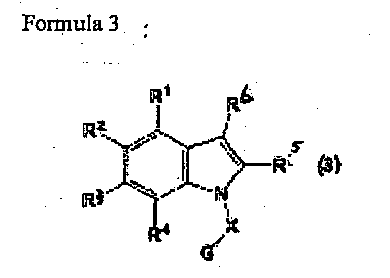 where R¹, R², R³, R⁴, X and G are the same as defined in Compound 1 above. R⁵ and R⁶ may be the same or different and they indicate an electron attractive group, an electron donating group or a hydrogen atom. 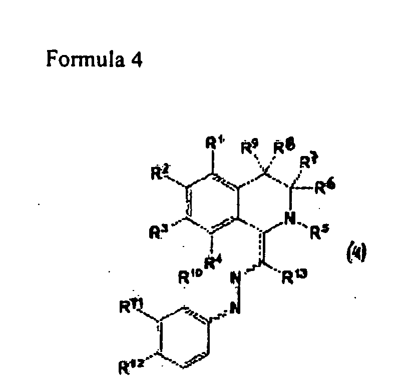 where R¹, R², R³, R⁴, R⁵, R⁶, R⁷, R⁸ and R⁹ are the same as defined above. R¹⁰, R¹¹, R¹² and R¹³ may be the same or different and represent an electron attractive group, an electron donating group or a hydrogen atom. The double single sided broken line indicates a single bond or a double bond. When the double single sided broken line indicates a double bond, a geometrical isomer is present for the wavy line part. There are no particular restrictions on where these geometrical isotropes are disposed and they may be independent of one another, or they may be E bodies or Z bodies. 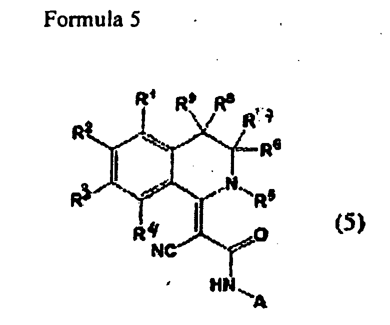 wherein A may be a hydrogen atom or Formula 6: 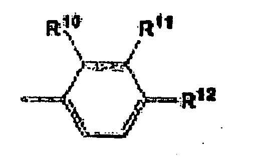 where R¹, R², R³, R⁴, R⁵, R⁶, R⁷, R⁸ and R⁹ are the same as defined above. R¹⁰, R¹¹, R¹² may be the same or different and indicate an electron attractive group, an electron donating group or a hydrogen atom. 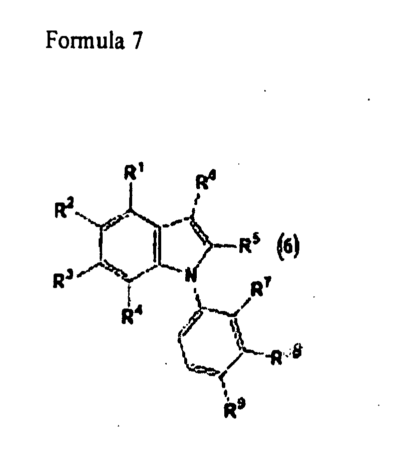 where R¹, R², R³, R⁴, R⁵ and R⁶ are the same as defined above. R⁷, R⁸ and R⁹ may be the same or different and indicate an electron attractive group, an electron donating group or a hydrogen atom. 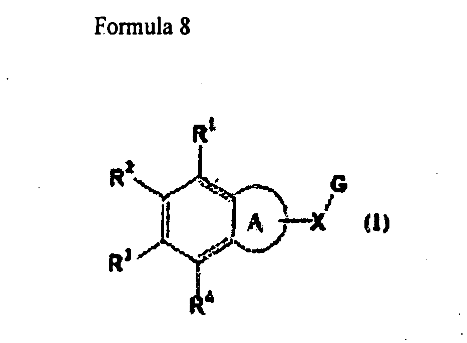 where R¹, R², R³ and R⁴ may be the same or different and represent an electron attractive group, an electron donating group or a hydrogen atom. By "electron donating group" is meant a substitution group which can donate electrons to a benzene ring; by "electron attractive group" is mean a substitution group which has the capacity to attract electon_{π} on a benzene ring. In addition, the electron donating group is defined as _{σ} < o and the electron attractive group is defined as _{σ} > o using Hammett's substitution group constant _{σ}. (Fundamental Organic Reaction Theory, Hashimoto Yasunobu, et al., Sankyo Publishing, 1997). 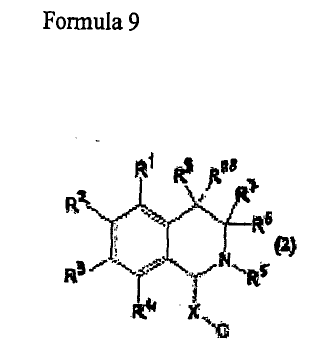 where R¹, R², R³, R⁴ and X and G are defined as above. R⁵, R⁶, R⁷, R⁸ and R⁹ may be the same or different and indicate an electron attractive group, an electron donating group or a hydrogen atom. 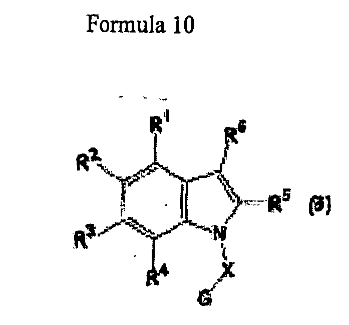 where R¹, R², R³, R⁴ and X and G are defined as above, R⁵ and R⁶ may be the same or different and represent an electron attractive group, an electron donating group or a hydrogen atom. 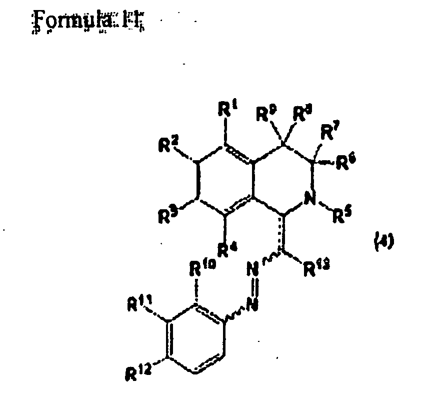 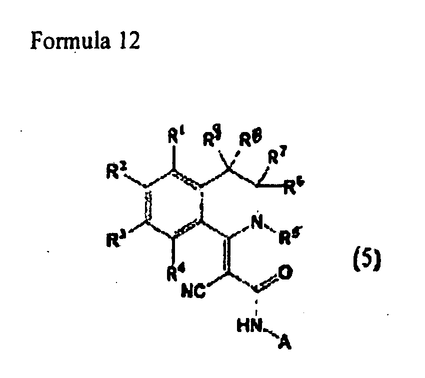 wherein A is a hydrogen atom or Formula 13: 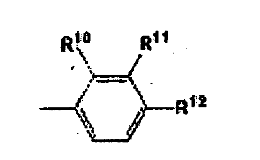 For Formulas 11 and 12, R¹ through R¹³ may be the same or different and they represent an electron attractive group, an electron donating group or a hydrogen atom. Specific examples include:
2- (4-acetyl-phenyl azo)-2-(3,3-dimethyl-3,4-dihydro-2H-isoquinoline-1-ylidene)-acetamide
2-(3-acetyl-phenyl azo)-2-(3,3-dimethyl-3,4-dihydro-2H-isoquinoline-1-ylidene)-acetamide
2-(4-bromo-phenyl azo)-2-(3,3-dimethyl-3,4-dihydro-2H-isoquinoline-1-ylidene)-acetamide
2-(3-bromo-phenyl azo)-2-(3,3-dimethyl-3,4-dihydro-2H-isoquinoline-1-ylidene)-acetamide
2-(4-chlor-phenyl azo)-2-(3,3-dimethyl-3,4-dihydro-2H-isoquinoline-1-ylidene)-acetamide
2-(3-chlor-phenyl azo)-2-(3,3-dimethyl-3,4-dihydro-2H-isoquinoline-1-ylidene)-acetamide
2-(3,3-dimethyl-3,4-dihydro-2H-isoquinoline-1-ylidene)-2-m-tolyl azo-acetamide
2-(3,3-dimethyl-3,4-dihydro-2H-isoquinoline-1-ylidene)-2-p-tolyl azo-acetamide
2-(3,3-dimethyl-3,4-dihydro-2H-isoquinoline-1-ylidene)-2-(4-methoxy-phenyl azo)-acetamide
2-(3,3-dimethyl-3,4-dihydro-2H-isoquinoline-1-ylidene)2-(3-methoxy-phenyl azo)-acetamide
2-(3,3-dimethyl-3,4-dihydro-2H-isoquinoline-1-ylidene)-2-(4-nitro-phenyl azo)-acetamide
2-(3,3-dimethyl-3,4-dihydro-2H-isoquinoline-1-ylidene)-2-(3-nitro-phenyl azo)-acetamide
2-(3,3-dimethyl-3,4-dihydro-2H-isoquinoline-1-ylidene)-2-(4-sulfamoyl-phenyl azo)-acetamide
2-(3,3-dimethyl-3,4-dihydro-2H-isoquinoline-1-ylidene)-2-(3,sulfamoyl-phenyl azo)-acetamide
2-(4-acetyl amino-phenyl azo)-2-(3,3-dimethyl-3,4-dihydro-2H-isoquinoline-1-ylidene)-acetamide
2-(3-acetyl amino-phenyl azo)-2-(3,3-dimethyl-3,4-dihydro-2H-isoquinoline-1-ylidene)-acetamide
2-(2-acetyl-phenyl azo)-2-(3,3-dimethyl-3,4-dihydro-2H-isoquinoline-1-ylidene)-acetamide
2-(3,3-dimethyl-3, 4-dihydro-2H-isoquinoline-1-ylidene)-2-phenyl azo-acetamide
(4-acetyl-phenyl azo)-(3,3-dimethyl-3,4-dihydro-2H-isoquinoline-1-ylidene)-ethyl acetate ester
2-(4-acetyl-phenyl azo)-2-(3,3-dimethyl-1,2,3,4-tetrahydro-isoquinoline-1-yl)-acetamide
2-(4-acetyl-phenyl azo)-2-(3,3-dimethyl-3,4-dihydro-2H-isoquinoline-1-ylidene)N-methyl-acetamide
2-(4-acetyl-phenyl azo)-2-(3,3-dimethyl-3,4-dihydro-2H-isoquinoline-1-ylidene)-N-phenyl-acetamide
2-(4-acetyl-phenyl azo)-2-(2,3,3-trimethyl-3,4-dihydro-2H-isoquinoline-1-ylidene)-acetamide
(4-acetyl-phenyl azo)-(3,3-dimethyl-3,4-dihydro-2H-isoquinoline-1-ylidene)-acetonitrile
2-(4-acetyl-phenyl azo)-2-(3,3-dimethyl-3,4-dihydro-2H-isoquinoline-1-ylidene)-N,N-dimethyl-acetamide
(4-acetyl-phenyl azo)-(3,3-dimethyl-3,4-dihydro-2H-isoquinoline-1-ylidene)-acetate
2-(2-acetyl-3,3-dimethyl-3,4-dihydro-2H-isoquinoline-1-ylidene)-2-(4-acetyl-phenyl azo)-acetamide
2-cyano-2-(3,3-dimethyl-3,4-dihydro-2H-isoquinoline-1-ylidene)-N-p-tolyl-acetamide
2-cyano-2-(3,3-dimethyl-3,4-dihydro-2H-isoquinoline-1-ylidene)-N-m-tolyl-acetamide
2-cyano-2-(3,3-dimethyl-3,4-dihydro-2H-isoquinoline-1-ylidene)-N-o-tolyl-acetamide
2-cyano-2-(3,3-dimethyl-3,4-dihydro-2H-isoquinoline-1-ylidene)-N-(4-methoxy-phenyl)-acetamide
2-cyano-2-(3,3-dimethyl-3,4-dihydro-2H-isoquinoline-1-ylidene)-N-(3-methoxy-phenyl)-acetamide
2-cyano-2-(3,3-dimethyl-3,4-dihydro-2H-isoquinoline-1-ylidene)-N-(4-nitro-phenyl)-acetamide
2-cyano-2-(3,3-dimethyl-3,4-dihydro-2H-isoquinoline-1-ylidene)-N-(3-nitro-phenyl)-acetamide
4-[2-cyano-2-(3,3-dimethyl-3,4-dihydro-2H-isoquinoline-1-ylidene-acetyl amino]-ethyl benzoate ester
3-[2-cyano-2-(3,3-dimethyl-3,4-dihydro-2H-isoquinoline-1-ylidene)-acetyl amino]-ethyl benzoate ester
2-cyano-2-(3,3-dimethyl-3,4-dihydro-2H-isoquinoline-1-ylidene)-N-phenyl-acetamide
2-cyano-2-(3,3-dimethyl-3,4-dihydro-2H-isoquinoline-1-ylidene)-N-(2,4-dimethylphenyl)-acctamide
2-cyano-2-(3,3-dimethyl-3,4-dihydro-2H-isoquinoline-1-ylidene)-acetamide 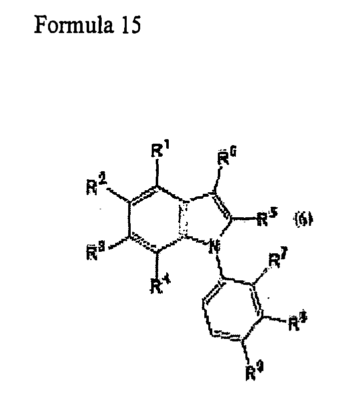 where R¹ through R⁹ are electron attractive group, an electron donating group or a hydrogen atom which may be the same or different. Of these, R¹ through R⁹ should be a group or an atom selected from a group made up of an alkyl group, an alkoxy group, a hydroxyl group, a nitro group, a nitrile group, an acetoxy group, an acetoxy alkyl group, a cyclic alkyl amino alkyl group which may include an oxygen atom, a dialkyl aminoalkyl group, a dialkyl amino vinyl group, a hydroxy alkyl amino alkyl group, an aryl aminovinyl group, an alkoxy carbonyl group, a halogen atom and a hydrogen atom. In addition, R¹ and R² should be a hydrogen atom; R³ should be a hydroxy group or an acetoxy group; R⁴ should be an acetoxy alkyl group, a cyclic alkyl aminoalkyl group which may contain an oxygen atom, a di-lower alkyl amino lower alkyl group, a hydroxy lower alkyl amino lower alkyl group or a hydrogen atom; R⁵ should be a lower alkyl group, a di-lower alkyl amino vinyl group or an aryl amino vinyl group; R⁶ should be a nitro group; R⁷, R⁸ and R⁹ should be a lower alkyl group, a lower alkoxy group or a hydrogen atom which may be the same or different.

Specific examples of the compound include:
2-methyl-3-nitro-1-phenyl-1H-indole-6-ol
1-(4-methoxy-phenyl)-2-methyl-3-nitro-1H-indole-6-ol
2-methyl-3-nitro-1-p-tolyl-1H-indole-6-ol
2-[2-(4-methoxy-phenyl amino)-vinyl] -3-nitro-1-p-tolyl-1H-indole-6-ol
1-(2-methoxy-phenyl)-2-methyl-3-nitro-1H-indole-6-ol
7-dimethyl amino methyl-2-(2-dimethyl amino-vinyl)-3-nitro-1-p-tolyl-1H-indole-6-ol
1-(4-methoxy-phenyl)-2-methyl-3-nitro-7-piperidine-1-yl methyl-1H-indole-6-ol-hydrochloride
2-(2-dimethyl amino-vinyl)-1-(4-methoxy-phenyl)-7-morpholine-4-yl methyl-3-nitro-1H-indole-6-ol
7-[(3-hydroxy-propyl amino)-methyl]-1-(4-methoxy-phenyl)-2-methyl-3-nitro-1H-indole-6-ol hydrochloride
7-dimethyl amino methyl-2-(2-dimethyl amino vinyl)-1-(4-methoxy-phenyl)-3-nitro-1H-indole-6-ol
7-diethyl amino methyl-1-(4-methoxy-phenyl)-2-methyl-3-nitro-1H-indole-6-ol
7-dimethyl amino methyl-2-methyl-3-nitro-1-p-tolyl-1H-indole-6-ol
1-(4-methoxy-phenyl)-2-methyl-3-nitro-7-piperidine-1-yl methyl-1H-indole-6-ol acetate 7-acetoxy methyl-2-methyl-3-nitro-1-p-tolyl-1H-indole-6-yl ester
2-(2-dimethyl amino vinyl)-1-(4-methoxy-phenyl)-3-nitro-7-piperidine-1-yl methyl-1H-indole-6-ol
7-dimethyl amino methyl-2-methyl-3-nitro-1-phenyl-1H-indole-6-ol
7-dimethyl aminomethyl-1-(4-methoxy-phenyl)-2-methyl-3-nitro-1H-indole-6-ol acetate 6-acetoxy-1-(4-methoxy-phenyl)-2-methyl-3-nitro-1H-indole-7-yl methyl ester
2-(2-dimethyl amino-vinyl)-3-nitro-1-p-tolyl-1H-indole-6-ol
2-(2-dimethyl amino-vinyl)-3-nitro-1-phenyl-1H-indole-6-ol
acetate 6-acetoxy-2-(2-dimethyl amino-vinyl)-1-(4-methoxy-phenyl)-3-nitro-1H-indole-7-yl methyl ester
1-(4-chlor-phenyl)-2-methyl-3-nitro-1H-indole-6-ol
acetate 2-(2-dimethyl amino-vinyl)-6-hydroxy-1-(4-methoxyl-phenyl)-3-nitro-1H-indole-7-il methyl ester
5-hydroxy-2-methyl-4,6-dinitro-1-phenyl-1H-indole-3-ethyl carbonate ester
7-[[bis-(2-hydroxy-ethyl)-amino]-methyl]-1-(4-methoxy-phenyl)-2-methyl-3 -nitro-1H-indole-6-ol
7-[[bis-(2-hydroxy-ethyl)-amino]-methyl]-2-methyl-3-nitro-1-p-tolyl-1H-indole-6-ol
7-dimethyl amino methyl-2-(2-dimethyl amino-vinyl)-3-nitro-1-phenyl-1H-indole-6-ol
2-(6-hydroxy-3-nitro-1-phenyl-1H-indole-2-yl methyl)-isothio urea
acetate 2-(N,N'-diphenyl-carbamimide yl sulfanyl methyl)-1-(4-methoxy-phenyl)-3-nitro-1H-indole-6-yl ester
acetate 6-acetoxy-1-(4-acetoxy-phenyl)-2-(dimethyl amino-vinyl)-3-nitro-1H-indole-7-yl methyl ester
2-(2-dimethyl amino-5-hydroxy-benzofurane-3-yl)-1-(4-methoxy-phenyl)-3-nitro-1H-indole-6-ol
2-(2-dimethyl amino-vinyl)-1-(4-methoxy-phenyl)-3-nitro-1H-indole-6-ol
5-bromo-1-(4-methoxy-phenyl)-2-methyl-3-nitro-1H-indole-6-ol
acetate 1-(4-methoxy-phenyl)-2-methyl-3-nitro-1H-indole-6-ol
7-dimethyl amino methyl-6-hydroxy-2-methyl-1-phenyl-1H-indole-3-carbonitrile
7-diethyl amino methyl-6-hydroxy-2-methyl-1-phenyl-1H-indole-3-carbonitrile
2-(2-dimethyl amino-5-hydroxy-benzofurane-3-yl)-6-methoxy-1-phenyl-1H-indole-3-carbonitrile
6-methoxy-2-methyl-1-phenyl-1H-indole-3-carbonitrile
2-(2-dimethyl amino-vinyl)-6-methoxy-1-phenyl-1H-indole-3-carbonitrile
5-bromo-6-hydroxy-1-(4-methoxy-phenyl)-2-methyl-1H-indole-3-carbonitrile
5,7-dibromo-6-hydroxy-1-(4-methoxy-phenyl)-2-methyl-1H-indole-3-carbonitile
6-hydroxy-1-(4-methoxy-phenyl)-2-methyl-1H-indole-3-carbonitrile
6-hydroxy-2-methyl-1-p-tolyl-1H-indole-3-carbonitrile
6-hydroxy-2-methyl-1-phenyl-1H-indole-3-carbonitrile
1H-furo [2,3-g] indole-3-acetate, 5-hydroxy-1-(4-methoxy phenyl)-2,8-dimethyl, ethyl ester
5-bromo-7-dimethyl aminomethyl-6-hydroxy-1-phenyl-2-phenyl sulfanyl methyl-1H-indole-3-ethyl acetate ester
6-hydroxy-2-methyl-1-phenyl-1H-indole-3-acetate
5-bromo-6-hydroxy-1-phenyl-2-phenyl sulfanyl methyl-1H-indole-3-ethyl acetate ester
6-acetoxy-5-bromo-2-methyl-1-phenyl-1H-indole-3-ethyl acetate ester
5-bromo-7-dimethyl amino methyl-6-hydroxy-1-phenyl-2-phenyl sulfanyl methyl-1H-indole-3-ethyl acetate ester.
6-acetoxy-5-bromo-2-bromomethyl-1-phenyl-1H-indole-3-ethyl acetate ester
6-acetoxy-2-bromo methyl-1-phenyl-1H-indole-3-ethyl acetate ester
6-acetoxy-2-methyl-1-phenyl-1H-indole-3-ethyl acetate ester
pyrrolo [2,3-f] [1,3] benzoxadine-3-acetate, 8-ethyl-1,7,8,9-tetrahydro-2-methyl-1-(4-nitrophenyl)-, ethyl ester
6-acetoxy-2-methyl-1-(2-trifluoromethyl-phenyl)-1H-indole-3-ethyl acetate ester
1-(2,4-dimethoxy-phenyl)-6-hydroxy-2-methyl-1H-indole-3-ethyl acetate ester
6-hydroxy-1-(4-methoxy-phenyl)-2-methyl-1H-indole-3-ethyl acetate ester
1-(4-ethoxy carbonyl-phenyl)-6-hydroxy-2-methyl-1H-indole-3-ethyl acetate ester
1-(4-cyano-phenyl)-6-hydroxy-2-methyl-1H-indole-3-ethyl acetate ester
6-hydroxy-2-methyl-1-(2-trifluoro methyl-phenyl)-1H-indole-3-ethyl acetate ester
6-hydroxy-2-methyl-1-(4-trifluoro methyl-phenyl)-1H-indole-3-ethyl acetate ester
6-hydroxy-2-methyl-1-(4-nitro-phenyl)-1H-indole-3-ethyl acetate ester
1-(4-bromo-phenyl)-6-hydroxy-2-methyl-1H-indole-3-ethyl acetate ester
1-(4-fluoro-phenyl)-6-hydroxy-2-methyl-1H-indole-3-ethyl acetate ester
6-hydroxy-2-methyl-1-(4-nitro-phenyl)-5,7-bis-piperidine-1-yl methyl-1H-indole-3-ethyl acetate ester
5,7-bis-dimethyl amino methyl-6-hydroxy-2-methyl-1-(4-nitro-phenyl)-1H-indole-3-ethyl acetate ester
6-hydroxy-2-methyl-1-(4-nitro-phenyl)-1H-indole-3-ethyl acetate ester
6-acetoxy-1-(4-chlor-phenyl)-2-methyl-1H-indole-3-ethyl acetate ester
7-dimethyl amino methyl-6-hydroxy-2-methyl-1-(4-nitro-phenyl)-1H-indole-3-ethyl acetate ester
7-dimethyl amino methyl-6-hydroxy-2-methyl-1-phenyl-1H-indole-3-ethyl acetate ester
6-hydroxy-2-methyl-1-p-tolyl-1H-indole-3-ethyl acetate ester
6-hydroxy-2-methyl-1-phenyl-1H-indole-3-ethyl acetate ester
1-(4-chlor-phenyl)-7-dimethyl amino methyl-6-hydroxy-2-methyl-1H-indole-3-ethyl acetate ester
1-(4-dimethyl amino-phenyl)-6-hydroxy-2-methyl-1H-indole-3-ethyl acetate ester
1-(2-chlor-phenyl)-6-methoxy-2-methyl-1H-indole-3-ethyl acetate ester
1-(2-chlor-phenyl)-6-hydroxy-2-methyl-1H-indole-3-ethyl acetate ester
6-acetoxy-1-(4-chlor-phenyl)-2-methyl-5,7-dinitro-1H-indole-3-ethyl acetate ester
1-(4-chlor-phenyl)-6-hydroxy-2-methoxy-2-methyl-5,7-dinitro-1H-indole-3-ethyl acetate ester
6-acetoxy-5,7-dibromo-1-(4-chlor-phenyl)-2-methyl-1H-indole-3-ethyl acetate ester
5,7-dibromo-1-(4-chlor-phenyl)-6-hydroxy-2-methyl-1H-indole-3-ethyl acetate ester
6-acetoxy-5-bromo-1-(4-chlor-phenyl)-2-methyl-1H-indole-3-ethyl acetate ester
5-bromo-1-(4-chlor-phenyl)-6-methoxy-2-methyl-1H-indole-3-ethyl acetate ester
1-(4-chlor-phenyl)-6-methoxy-2-methyl-1H-indole-3-acetate
1-(4-chlor-phenyl)-6-methoxy-2-methyl-1H-indole-3-ethyl acetate ester
1-(4-chlor-phenyl)-6-hydroxy-2-methyl-1H-indole-3- ethyl acetate ester

Examples of the lower alkyl group represented by R are as follows: methyl, ethyl, n-propyl, isopropyl, n-butyl, isobutyl, sec-butyl, tert-butyl, n-pentyl, isopentyl, neopentyl, 1-methyl propyl, n-hexyl, isohexyl, 1,1-dimethyl butyl, 2,2-dimethyl butyl, 3,3-dimethyl butyl, 3,3-dimethyl propyl, 2-ethyl propyl and the like. Methyl is especially suitable. Examples of the lower alkoxy group represented by R are as follows: methoxy, ethoxy, propoxy, isopropoxy, butoxy, sec-butoxy, tert-butoxy, pentoxy, hexyloxy, heptyloxy, octyloxy and the like. Methoxy is especially suitable.

Examples of the halogen atom represented by R are as follows: fluorine, chlorine, bromine, iodine and the like but chlorine or bromine are especially suitable. Examples of the lower acyl group represented by R are as follows: formyl, acetyl, propionyl, butyryl and the like and acetyl is especially suitable. Examples of the lower alkyl group which may form the cyclic structure represented by R are as follows: cyclobutyl, cyclopentyl, cyclohexyl, cycloheptyl, cyclooctyl and the like. Cyclopentyl, cyclohexyl and cycloheptyl are especially suitable.

Examples of the lower alkoxy carbonyl represented by R are as follows: methoxy carbonyl, ethoxy carbonyl, propoxy carbonyl and the like, with methoxy carbonyl or ethoxy carbonyl being especially suitable. Examples of the amino carbonyl represented by R are as follows: -CONR₂ (R is a hydrogen atom which may be the same or different; it represents the lower alkyl group illustrated previously, and a phenyl group which may have a substitution group).

Examples of the secondary aminocarbonyl group represented by R are -CONHR (indicates a lower alkyl group illustrated previously and a phenyl group which may have a substitution group). In addition, the tertiary amino carbonyl group may be -CONR₂ (R represents the lower alkyl group which was illustrated previously which may be the same or different, and a phenyl group which may have a substitution group). Examples of the amino alkyl group represented by R are: -(CH₂)ₙ -NR₂ (where n is an integer from 1 to 8 and preferably 1). R is a hydrogen atom, a lower alkyl group, or a lower alkyl group which may form a cyclic structure which may be the same or different (1 to 3 hetero atoms may be included in the nitrogen and oxygen in the cyclic structure), and a phenyl group which may have a substitution group) and the like.

An example of the acetoxy alkyl represented by R is: -(CH₂)ₙ -Oac (where n is an integer from 1 to 8) and n is preferably 1.

Other specific examples include Compounds of formulas 17-28 below.

### 3,3-dimethyl-1,2,,3,4-tetrahydroisoquinolinidene-1-acetamide (formula 17)

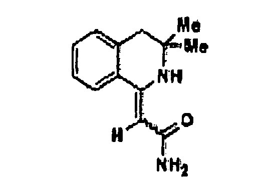

### 2-(4-acetyl-phenyl azo)-2-(3,3-dimethyl-3,4-dihydro-2H-isoquinoline-1-ylidene)-acetamide (formula 18)

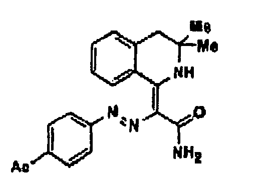

### 2-(3-acetyl-phenyl azo)-2-(3,3-dimethyl-3,4-dihydro-2H-isoquinoline-1-ylidene)-acetamide (formula 19)

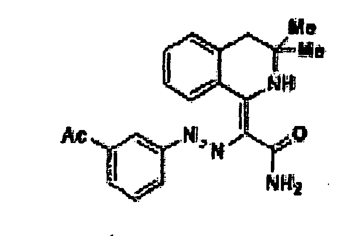

### 2-(4-acetyl-phenyl azo)-2-(2,3,3-trimethyl-3,4-dihydro-2H-isoquinoline-1-ylidene)-acetamide (formula 20)

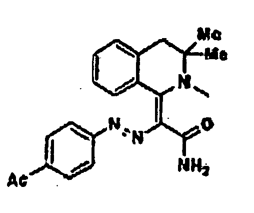

### 2-(2-acetyl-3,3-dimethyl-3,4-dihydro-2H-isoquinoline-1-ylidene)-2-(4-acetyl-phenyl azo)-acetamide (formula 21)

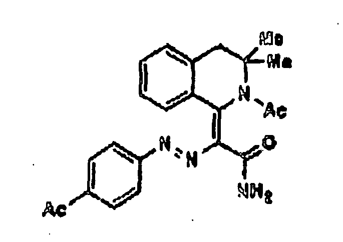

### (4-acetyl-phenyl azo)-(3,3-dimethyl-3,4-dihydro-2H-isoquinoline-1-ylidene)-acetonitrile (formula 22)

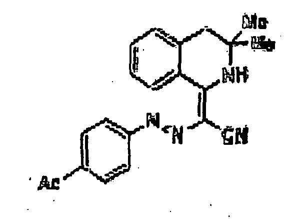

### (4-acetyl-phenyl azo)-(3,3-dimethyl-3,4-dihydro-2H-isoquinoline-1-ylidene)-acetate (formula 23)

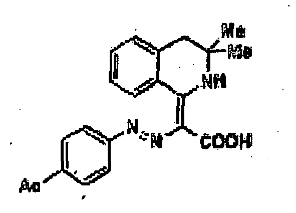

### (4-acetyl-phenyl azo)-(3,3-dimethyl-3,4-dihydro-2H-isoquinoline-1-ylidene)-ethyl acetate ester (formula 24)

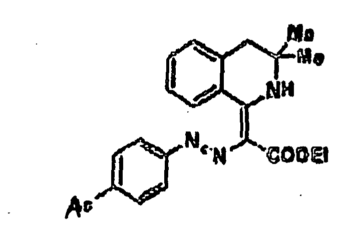

### 2-(4-acetyl-phenyl azo)-2-(3,3-dimethyl-3,4-dihydro-2H-isoquinoline-1-ylidene)-N-methyl-acetamide (formula 25)

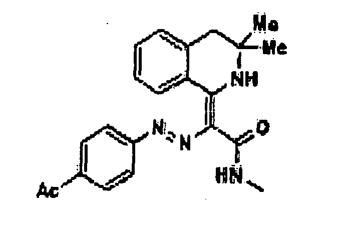

### 2-(4-acetyl-phenyl azo)-2-(3,3-dimethyl-3,4-dihydro-2H-isoquinoline-1-ylidene)-N,N-dimethyl-acetamide (formula 26)

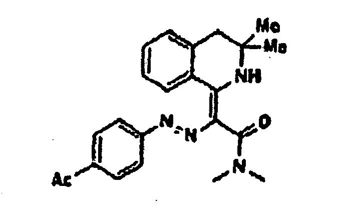

### 2-(4-acetyl-phenyl azo)-2-(3,3-dimethyl-3,4-dihydro-2H-isoquinoline-1-ylidene)-N-phenyl-acetamide (formula 27)

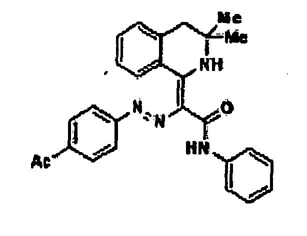

### 2-(4-acetyl-phenyl azo)-2-(3,3-dimethyl-1,2,3,4-tetrahydro-isoquinoline-1-yl)-acetamide (formula 28)

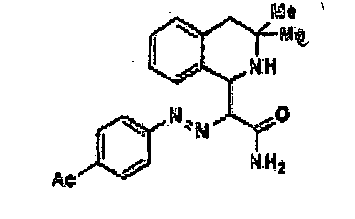

Other examples of compounds include:
2-cyano-2-(3,3-dimethyl-3,4-dihydro-2H-isoquinoline-1-ylidene)-N-p-tolyl-acetamide
2-(4-acetyl-phenyl azo)-2-(3,3-dimethyl-3,4-dihydro-2H-isoquinoline-1-ylidene)-acetamide
2-(3-chlor-phenyl azo)-2-(3,3-dimethyl-3,4-dihydro-2H-isoquinoline-1-ylidene)-acetamide
2-(3,3-dfinethyl-3,4-dihydro-2H-isoquinoline-1-ylidene)-2-(3-methoxy-phenyyl azo)-acetamide
2-(3,3-dimethyl-3,4-dihydro-2H-isoquinoline-1-ylidene)-2-(3-nitro-phenyl azo)-acetainide
2-(3,3-dimethyl-3,4-dihydro-2H-isoquinoline-1-ylidene)-2-(4-tolyl-azo-phenyl azo)-acetamide
2-(3-acetyl-phenyl azo)-2-(3,3-dimethyl-3,4-dihydro-2H-isoquinoline-1-ylidene)-acetamide
2-(3,3-dimethyl-3,4-dihydro-2H-isoquinoline-1-ylidene)-2-in-tolyl-azo-acetamide 2-methyl-3-nitro-1-phenyl-1H-indole-6-ol
1-(4-methoxy-phenyl)-2-methyl-3-nitro-1H-indole-6-ol 2-methyl-3-nitro-1-p-tolyl-1H-indole-6-ol
2-[2-(4-methoxy-phenyl amino)-vinyl]-3-nitro-1-p-tolyl-1H-indole-6-ol 1-(2-methoxy-phenyl)-2-methyl-3-nitro-1H-indole-6-ol
7-dimethyl amino methyl-2-(2-dimethyl amino-vinyl)-3-nitro-1-p-tolyl-1H-indole-6-ol
1-(4-methoxy-phenyl)-2-methyl-3-nitro-7-piperidine-1-yl methyl-1H-indole-6-ol
2-(2-dimethyl amino-vinyl)-1-(4-methoxy-phenyl)-7-morpholine-4-yl methyl-3-nitro-1H-indole-6-ol
7-[(3-hydroxy-propyl amino)-methyl]-1-(4-methoxy-pbenyl)-2-methyl-3-nitro-1H-indole-6-ol
7-dimethyl amino methyl-2-(2-dimethyl amino-vinyl)-1-(4-methoxy-phenyl)-3-nitro-1H-indole-6-ol
7-dimethyl amino methyl-1-(4-methoxy-phenyl)-2-methyl-3-nitro-1H-indole-6-ol
7-dimethyl amino methyl-2-methyl-3-nitro-1-p-tolyl-1H-indole-6-ol
1-(4-methoxy-phenyl)-2-methyl-3-nitro-7-piperidine-1-yl methyl-1H-indole-6-ol
acetate 7-acetoxy methyl-2-methyl-3-nitro-1-p-tolyl-1H-indole-6-ol ester
2-(2-dimethyl amino-vinyl)-1-(4-methoxy-phenyl)-3-nitro-7-piperidine-1-yl methyl-1H-indole-6-ol
7-dimethyl amino methyl -2-methyl-3-nitro-1-phenyl-1H-indole-6-ol
7-dimethyl amino methyl-1-(4-methoxy-phenyl)2-methyl-3-nitro-1H-indole-6-ol acetate 6-acetoxy-1-(4-methoxy-phenyl)-2-methyl-3-nitro-1H-indole-7-yl methyl ester
2-(2-dimethyl amino-vinyl)-3-nitro-1-p-tolyl-1H-indole-6-ol
2-(2-dimethyl amino-vinyl)-3-nitro-1-phenyl-1H-indole-6-ol
acetate 6-acetoxy-2-(2-dimethyl amino-vinyl)-1-(4-methoxy-phenyl)-3-nitro-1H-indole-7-yl methyl ester
1-(4-chlor-phenyl)-2-methyl-3-nitro-1H-indole-6-ol
acetate 2-(2-dimethyl amino-vinyl)-6-hydroxy-1-(4-methoxy-phenyl)-3-nitro-1H-indole-7-yl methyl ester
5-hydroxy-2-methyl-4,6-dinitro-1-phenyl-1H-indole-3-ethyl carboxylate ester

Such compounds can be tested as described in the Examples herein for their suitability for practicing the methods disclosed herein.

It should be understood that the examples and embodiments described herein are for illustrative purposes only and that various modifications or changes in light thereof will be suggested to persons skilled in the art.

### EXAMPLES

### The following examples do not fall within the scope of the presently claimed invention but are helpful in undertanding the invention by demonstrating the activing of a compound that may be identified using the method of the invention. Experimental Procedures used in Examples 1-7

Cell culture. ESCs, D3ES (ATCC CRL-1934) were maintained on Mitomycin C treated MEFs in mouse ESC medium containing DMEM (Invitrogen) supplemented with 15% FBS (Invitrogen), 0.1mM MEM nonessential amino acids (Invitrogen), 0.1mM 2-mercaptoethanol (Sigma), 2mM L-glutamine (Invitrogen) and 1,000 U/ml LIF (CHEMICON). To remove MEFs, cells were collected by trypsinization and plated on gelatin-coated culture dishes for 20 min. Non adherent cells consisting mainly of ESCs were replated on gelatin-coated culture dishes again. Non-adherent cells were used for further experiments. P19 cells were cultured according to conditions recommended by the ATCC. Cells were incubated at 37°C in a 5% CO₂ incubator. Purified Wnt3a was purchased (R&D Systems).

Compound Screens. For the small molecule screen, alkaline phosphatase activity of ESCs was determined. ESCs were plated into 96-well tissue culture plates (Falcon) at a density of 316-1,000 cells per well in ESC medium without LIF, Compounds were added at a final concentration of 4 µg/ml. Cells were treated with compounds for 7 days and test cell populations were washed with PBS and 100µl of p-nitrophenyl phosphate solution (MOSS Inc.) was added. The absorbance at 405nm was measured by spectrophotometer (Spectramax, Molecular Devices).

Flow Cytometry. Analysis of SSEA-1 expression of ESCs was performed by flow cytometry according to Zandstra et al. (35). The test cell population was washed in ice cold Hanks balanced salt solution (Invitrogen) containing 2%FCS (HF) and resuspended for 10min in HF containing anti-mouse CD16/CD32 monoclonal antibody at 1 µg/100µl (Phamingen) to block nonspecific binding. Blocked cells were then incubated at 1x10⁷ cells/ml for 40min on ice with anti-SSEA-1,(Kyowa Medex) followed by FITC-Goat anti Mouse IgM antibody (ZYMED). Cells to be analyzed for their SSEA-1 expression were then washed twice in HF with 2µg/ml propidium iodide (Dojindo) added into the final wash. The cells were then resuspended in HF for analysis on a FACS Calibur (Becton Dickinson).

NIH-3T3 cells (wt), NIH-3T3 CBP(+/-) cells and NIH-3T3 p300(+/-) cells were transfected as described in (26). Briefly, cells were transfected with TOPFLASH or FOPFLASH, using Fugene6 (Roche Molecular Biochemicals). Transfection efficiencies were normalized with pRL-null luciferase plasmid. Luciferase assays were performed with the DUAL-Luciferase Reporter Assay System (Promega).

Real-time RT-PCR. Total RNA was isolated and reverse-transcribed using SuperScript III (Invitrogen). Real-time RT-PCR (Sybr Green; BioRad) was performed using with the gene-specific primers:
Nanog-F agggtctgctactgagatgctctg (SEQ ID NO:5)
Nanog-R caaccactggtttttctgccaccg (SEQ ID NO:6)
GAPDH-F ggtgaaggtcggtgtgaacgga (SEQ ID NO:7)
GAPDH-R tgttagtggggtctcgctcctg (SEQ ID NO:8)

In certain experiments, 2 µ g of extracted total RNA was reverse-transcribed using SuperScript III RT-PCR system (Invitrogen) according to the manufacturer's protocol. 1 µl of cDNA was amplified by PCR with one or more of the gene-specific primers of SEQ ID NOs:5-18 using optimized PCR cycles to obtain amplified reactions in the linear range.

| | |
|---|---|
| Oct-3/4-F | ggcgttctctttggaaaggtgttc (SEQ ID NO:9) |
| Oct-3/4-R | ctcgaaccacatccttctct (SEQ ID NO:10) |
| Rex-1-F | gtcttatcgatgctggagtg (SEQ ID NO:11) |
| Rex-1-R | aaagctcttctcgcagccat (SEQ ID NO:12) |
| Sox-2-F | gcatgtcctactcgcagcag (SEQ ID NO:13) |
| Sox-2-R | gctgatcatgtcccggaggt (SEQ ID NO:14) |
| C-Myc-F | accaacagcaactatgacctc (SEQ ID NO:15) |
| C-Myc-R | aaggacgtagcgaccgcaac (SEQ ID NO:16) |
| MDR-1-F | tgcttatggatcccagagtga (SEQ ID NO:17) |
| MDR-1-R | ttggtgaggatctctccgcgt (SEQ ID NO:18) |

Transfection and Luciferase assay. ESCs were cultured in 96-well cell culture dishes coated with a 0.1% aqueous gelatin solution and transfected with 0.2µg/well of pSTAT3-TA-Luc (CLONTECH), using Lipofectamine 2000 (Invitrogen). After 6 hours, cells were washed and exposed to either IQ-1 at the indicated doses, or LIF, for 24 hours. Transfection efficiencies were normalized with pRL-null luciferase plasmid. Luciferase assays were performed with the DUAL-Luciferase Reporter Assay System (Promega).

In certain experiments, NIH-3T3 cells (wt), NIH-3T3 CBP(+/-) cells and NIH-3T3 p300(+/-) cells were transfected as described in (26). Briefly, cells were transfected with TOPFLASH or FOPFLASH, using Fugene6 (Roche Molecular Biochemicals). Transfection efficiencies were normalized with pRL-null luciferase plasmid. Luciferase assays were performed with the DUAL-Luciferase Reporter Assay System (Promega).

Detection of CBP, p300, and phospho-serine89-p300 in P19 cells. P19 cells exposed to either Wnt3A or vehicle control, after which IQ-1 was added to a final concentration of 10µM. Control DMSO was 0.025%. Cells were incubated for 24 hours. At the end of this incubation period, cells were washed, lysed and subjected to SDS-PAGE. CBP and p300 were detected using the rabbit polyclonal antibody (A-22)(1:5000); and (C-20)(1:5000) (Santa Cruz), respectively. Phospho-serine89-p300 was detected using custom antisera at a dilution of 1:100.

Co-immunonrecipitation of β-catenin with CBP or p300 in P19 cells. Co-iimunoprecipitation was performed as described in (27). Briefly, P19 cells were treated with 10µm ICG-001 or 10 µM IQ-1 (control DMSO was 0.025%) for 24 hours, after which they were washed and lysed using. Nuclear fraction was isolated and precleared with antibodies to CBP(A-22) or p300(N-15). β-catenin was detected using a mouse monoclonal antibody (Becton Dickinson) at a dilution of 1:5000.

Affinity of *in vitro* phosphorylated p300 with β-catenin. Recombinant p300 (1-110 aa) fused to an HA-tag were incubated with PKCα in kinase buffer (20 mM Hepes, pH 7.4, 10 mM magnesium acetate, 1 mM dithiothreitol, 100 µM ATP). Co-immunoprecipitation was carried out in P19 lysates mixed with the *in vitro* phosphorylated p300 using an HA-tag antibody, p300 was detected using the rabbit polyclonal antibody (C20), 1:5000. β-catenin was detected using a mouse monoclonal antibody (Becton Dickinson) at a dilution of 1:5000

Affinity Purification of Target Proteins. P19 cells were cultured to 90-100% confluency. Cells were lysed in protein-binding buffer [PBB, 20 mM Hepes, pH 7.9/100mM NaCL/0.5 mM EDTA/0.5% Nonidet P-40/6mM MgCl2/5 mM 2-mercaptoethanol/0ne tablet of Complete protease inhibitor mixture (Roche Molecular Biochemicals)]. Biotinylated IQ-1 was bound overnight at room temperature to a 50% slurry of streptavidin-agarose beads (Amersham Phannacia) in buffer containing 50% DMSO and 50% PBB. Beads were washed to remove unbound IQ-1 and then incubated with whole cell lysates. Proteins eluted by boiling in SDS were Coomassie stained to detect target proteins, or immunoblotted.

Zebrafish experiments. Wild-type (AB) zebrafish strain at 1-cell stage were treated with IQ-1 at a final concentration of 1 µM in embryo medium for 24h. Control zebrafish embryos were incubated at an equivalent concentration of DMSO. At the end of this 24h treatment, embryos were manually dechorionated and imaged. Zebrafish embryos were maintained at 28°C. Results shown are representative of those obtained from at least 10 embryos for each group, from 2 independent experiments.

Immunocytochemistry and Antibodies. Cells were fixed for 20 min with 4% paraformaldehyde in PBS. Immunostaining was carried out using standard protocols. Primary antibodies were used at the following dilutions: anti α-fetoprotein mouse monoclonal (R&D systems, 10µg/ml), anti-Actin, Smooth muscle Ab-1 mouse monoclonal (LAB VISION, 1:1), anti-GATA4 mouse monoclonal (SANTA CRUZ, 1:100), anti- MAP(microtubule associated protein)2 mouse monoclonal (Chemicon, 1:200), βIII-tubulin mouse monoclonal (Chemicon, 1:200), anti-Oligodendrocyte mouse monoclonal (Chemicon, 1:1,000). Secondary antibodies were Alexa Fluor 488 or 594 goat anti-mouse IgG (H+L)(Invitrogen, 1:200). Cells were imaged using an Olympus IX 70 microscope with x 40-200 magnification.

### EXAMPLE 1

### PR72/130 AND ES CELL PROLIFERATION

The small molecule IQ-1 (Asahi Kasei Pharma, Figure 1) selectively increased β-catenin's usage of CBP as a coactivator at the expense of the use of p300, thereby maintaining the embryonic (ES) cells in an undifferentiated state. The combination of Wnt3a and IQ-1 allowed for proliferation and maintenance of pluripotency as judged by the expression of Oct4, Nanog and Rex1, whereas IQ-1 alone was not sufficient to cause proliferation and maintain pluripotency of ES cells.

The present example relates to the identification of the molecular target of IQ-1. To identify the molecular target of IQ-1, whole cell lysates from P19 embryonic carcinoma cells were treated with biotinylated IQ-1. Compared to a control biotinylated compound (Fig. 3A, lane 2), biotinylated IQ-1 selectively bound three proteins (Fig. 3A, lane 3). The two bands at 72 and 130 kDa were identified by mass spectral sequencing as the differentially spliced regulatory subunits PR72/130 of the serine/threonine protein phosphatase, PP2A. This was subsequently confirmed by immunoblotting (Fig. 3B).

The fact that IQ-1 selectively binds to these proteins correlates well with the effects seen on the modulation of Wnt signaling, as it has previously been shown that PR72/130 interacts with the protein Naked cuticle, a component of the Wnt signaling pathway, thereby regulating Wnt signaling (Creyghton, M.P. et al., Genes and Dev 19:376-386 2005). Using morpholinos in xenopus embryos, it was also demonstrated that PR72, like Naked cuticle (Nkd), is a negative regulator of Wnt/β-catenin signaling and involved in the switch to non-canonical convergent extension (Creyghton et al.).

### EXAMPLE 2

### IQ-1 MAINTAINED THE UNDIFFERENTIATED STATE OF ESCs

Murine ESCs (D3 ES) were screened with a chemical library (Asahi Kasei) to identify compounds that enhanced Alkaline Phosphatase (AP) production, a marker of undifferentiated ESCs (12). From this screen, IQ-1 (MW= 362.42, Fig. 1A) was identified, which dose dependently increased AP activity (Fig. 1B), in media containing 15% FCS without the addition of exogenous leukemia inhibitory factor (LIF). Treatment with IQ-1 resulted in enhanced expression of the undifferentiated ESC marker, Stage Specific Embryonic Antigen 1 (SSEA-1) in a dose dependent fashion (Fig. 1C).

IQ-1 allowed for long term expansion of ESCs in culture without MEFs and without the addition of exogenous LIF. Mouse D3 ESCs were cultured in media containing 15% FCS plus 4µg/ml IQ-1 for 65 days. The ESCs continued to proliferate at a steady rate with an approximate 2 log increase every ten days (Fig. 1D).

### EXAMPLE 3

### IQ-1 MAINTAINED MURINE ESC SELF-RENEWAL INDEPENDENTLY OF LIF

The cytokine LIF, by activating the Stat3 signal transduction pathway, maintains murine ESC symmetrical self-renewal and blocks differentiation (13, 14, 15). LIF did not maintain human ESC self-renewal (16, 17). Nanog is a divergent homeoprotein pluripotency sustaining factor for ESCs (18, 19). Nanog has been shown to act in parallel with LIF-driven stimulation of Stat3 to drive ESC self-renewal. Additionally, elevated expression of Nanog is sufficient for clonal expansion of ESCs and maintenance of expression of the key stem cell transcription factor Oct4, in the absence of Stat3 activation (19).

To further investigate the mechanism of action of IQ-1, the effects of IQ-1 on Nanog expression were determined. Whereas feeder-free ESCs treated with LIF only slightly increased Nanog levels, IQ-1 significantly increased and maintained Nanog expression in culture as judged by real time RT-PCR (Fig. 2A). Removal of IQ-1 from culture led to a precipitous drop in Nanog level (Fig. 2B). Loss of Nanog expression has been previously correlated with ESC differentiation (18, 19). To further confirm that the effects of IQ-1 were not mediated via Stat3 signaling, a Stat3/luciferase reporter gene construct was utilized. While IQ-1 significantly elevated Nanog expression, it did not affect Stat3/luciferase expression unlike LIF, which as anticipated, elicited a significant response (Fig. 2C). From this Example, it can be concluded that the affects of IQ-1 on the maintenance of murine ESC pluripotency are independent of the LIF/Stat3 pathway.

### EXAMPLE 4

### IQ-1 MODULATES WNT SIGNALING VIA INTERACTION WITH PR72/130.

This example was performed to examine the effects of IQ-1 on "non-canonical" Wnt signaling by looking at convergent extension during zebrafish (danio rerio) development. Zebrafish embryos treated with 1µM IQ-1 showed significant developmental defects (Fig. 3C). In particular a shortened tail, consistent with inhibition of convergent extension and similar to the effects of PR72 morpholinos, was observed (25). From this Example and Example 1, it can be concluded that the molecular target of IQ-1 is PR72/130. The interaction of IQ-1 with PR72/130 results in the disruption of the PR72/130/Nkd complex, and inhibits "negative" regulation of canonical Wnt/β-catenin signaling and a concurrent switch to "non-canonical" convergent extension.

### EXAMPLE 5

### IQ-1 MAINTENANCE OF ESCs IS WNT/B-CATENIN/CBP DEPENDENT

Wnt/β-catenin signaling has been demonstrated to inhibit neuronal differentiation and maintain pluripotency in stem cells (1-3) and is critical for the expansion of progenitors (4). However, Wnt/β-catenin signaling is also required for neural differentiation of ESCs and neural stem cells (5-6). Using ICG-001, a recently characterized specific antagonist of the β-catenin/CBP interaction, a model was developed to explain the divergent activities of Wnt/β-catenin signaling (7, 8, 26, 27). The key feature of this model is that β-catenin/CBP-mediated transcription is critical for "stem/progenitor" cell proliferation, whereas a switch to β-catenin/p300-mediated transcription is critical to initiate a differentiative program with a more limited proliferative capacity.

The Wnt/β-catenin reporter constructs TOPFLASH and FOPFLASH in wild-type NIH-3T3 cells (wt), NIH-3T3 CBP(+/-) cells and NIH-3T3 p300(+/-) cells were used to determine whether IQ-1, by targeting the PR72/130 subunit with PP2A, was selectively increasing β-catenin's usage of CBP as a coactivator at the expense of p300, thereby maintaining the ESCs in the undifferentiated state (Fig. 4A). A point mutant constitutively translocating β-catenin (pt-mut β-cat) was used to stimulate reporter activity (28). Although IQ-1 did not affect TOPFLASH or FOPFLASH activity in either the wt or CBP (+/-) cells, a 2-3 fold increase in TOPFLASH activity was observed in the p300 (+/-) cells (Fig. 6). This suggests that the effects of IQ-1 are coactivator specific and dependent on the expression level of p300.

To directly evaluate the effects of IQ-1 on β-catenin coactivator usage, co-immunoprecipitation with antibodies to either CBP or p300 was performed, followed by immunoblotting for coactivator-associated β-catenin utilizing P19 embryonic carcinoma cells treated with Wnt3a in the presence or absence of IQ-1. IQ-1 caused a dramatic increase in the relative amount of β-catenin associated with CBP compared to cells treated with Wnt3a and either DMSO or the CBP/β-catenin antagonist ICG-001. ICG-001, which induces cellular differentiation (7), significantly enhanced the p300/β-catenin interaction at the expense of the CBP/β-catenin interaction (Fig. 4B).

### EXAMPLE 6

### IQ-1 INDIRECTLY DECREASES THE PHOSPHORYLATION OF P300 SER89 AND THEREBY THE B-CATENIN/P300 INTERACTION

It is known that signaling through the "non-canonical" Wnt pathway can increase PKC activity (29) and that Ser89 of p300 can be phosphorylated in a PKC-dependent fashion (30). The present example was performed to evaluate the effects of PKCα phosphorylation of p300 Ser89 on the binding of p300 to β-catenin. Recombinant p300 (1-110aa) was phosphorylated with purified PKCα. The *in vitro* phosphorylated p300 was then added to cell lysates from P19 cells and the β-catenin/p300 complexes were co-immunoprecipitated. Prior phosphorylation by PKCα enhanced the p300/β-catenin interaction (Fig. 4C, compare lane 2 to lane 1), To determine if the enhanced interaction was dependent on p300 Ser89 phosphorylation by PKCα, the serine residue was mutated to alanine in the recombinant p300 fragment. Mutagenesis of Ser89 to Ala89 abrogated the PKCα-dependent increase in binding to β-catenin (Fig. 4C, compare the difference between lanes 3 and 4 to lanes 1 and 2). This indicates that phosphorylation of p300 Ser89 enhances the affinity of the β-catenin/p300 interaction.

To determine the physiological relevance of this phosphorylation-dependent mechanism for increasing the β-catenin/p300 interaction in cells, P19 cells were exposed to purified Wnt3a and either treated with IQ-1 or DMSO control. As judged by immunoblotting, IQ-1 treatment of Wnt3a stimulated P19 cells caused a dramatic decrease in the phosphorylation level of p300 Ser89 (Fig. 4D Top, compare lanes 2 and 3) whereas the total amount of p300 was not affected by IQ-1 (Fig. 4D Middle, compare lanes 2 and 3). Based on these results, it can be concluded that IQ-1 by negatively regulating the phosphorylation of p300 Ser89 thereby decreases the affinity of the β-catenin/p300 interaction and increases β-catenin/CBP usage.

### EXAMPLE 7

### LONG TERM MAINTENANCE OF ESCs IN SERUM FREE MEDIA CONTAINING IQ-1 AND WNT3A

Real-time RT-PCR of P19 cells treated with Wnt3a and IQ-1 revealed increased expression of "stem/progenitor" markers including *Oct4, Sox2* (31) and *MDR*-*1* (32) and a decrease in *c-myc* (33) expression compared to Wnt3a + DMSO treated cells (Table 1). Addition of purified Wnt3a (100ng/ml) to 15% KSR (serum replacement media) in conjunction with 4µg/ml of IQ-1 was sufficient to increase alkaline phosphatase levels and maintain ESC pluripotency long term (48 days), similar to results obtained with IQ-1 and 15% FCS (Fig. 7). Neither Wnt3a nor IQ-1 alone was sufficient to maintain the undifferentiated status of ESCs in KSR media (Fig. 8). Wnt3a alone induced proliferation, but was not sufficient to maintain the expression of the stem cell markers Oct4, Nanog or Rexl. However, the combination of Wnt3a and IQ-1 allowed for proliferation and maintenance of pluripotency as judged by the expression of Oct4, Nanog and Rexl (Fig. 9A, B, and C). Treatment of ESCs with IQ-1 and Wnt3a maintained the pluripotency of ESCs as judged by their ability to form embryoid bodies after day 48 (Fig. 5A, left panel) and the ability to differentiate to all three germ layer-derived tissues (Fig. 5B). Removal of IQ-1 led to a rapid (within 3 days) loss of pluripotency and the inability to form embryoid bodies (Fig. 5*A*, right panel). Based on this example, it can be concluded that IQ-1 by increasing Wnt/β-catenin/CBP-dependent signaling and preventing the switch to Wnt/β-catenin/p300 mediated transcription was sufficient to maintain long term murine ESC pluripotency.

**Table 1**

| | ΔΔCTIQ-1 |
|---|---|
| Sox-2 | 4.31 |
| MDR-1 | 1.69 |
| Oct4 | 0.39 |
| c-myc | -2.01 |

In Table 1, above, the effects of IQ-1 treatment on genes associates with stem cell maintenance are shown, as measured by RT-PCR. The values are represented as ΔΔCT from the control gene β-actin.

These Examples demonstrate that IQ-1 in conjunction with purified Wnt3a is sufficient to maintain murine ESC proliferation and pluripotency for extended periods of time in culture (at least 48 days) in the absence of serum. IQ-1 plus Wnt3a upregulated the expression of the transcription factors *Oct4* and *Sox2*, which are critical to ESC maintenance. Recently, Boyer et al. demonstrated that Oct4 and Sox2 co-occupy the promoters/enhancers of a substantial portion of the genes required for the maintenance of human ESCs (31). As shown in the Examples, IQ-1 downregulates the expression of *c-myc*. *C-myc* appears to be a critical player in the balance between stem cell self-renewal and differentiation and is increased upon differentiation (33).

Using an affinity chromatography approach, it was determined that the molecular target(s) of IQ-1 are the differentially spliced regulatory subunits PR72/PR130 of the protein phosphatase PP2A. This is extremely interesting in that PR72 interacts with the protein Nkd (25), a Wnt-inducible antagonist of "canonical" Wnt/β-catenin signaling (24). Similar to the results observed with PR72 morpholinos (25), IQ-1 had dramatic effects on "non-canonical" convergent extension during zebrafish embryonic development. The question of the roles of the two splice variants PR72 and PR130 in the switch from "canonical" to "non-canonical" wnt signaling appears to be quite complex. Recently, Bernards *et al*. showed that unlike PR72, PR130 can antagonize some of the effects of Nkd in a variety of assays. However, PR130 morphilinos affected somite development and tail formation in xenopus embryos (34). Therefore, the effects of IQ-1 on zebrafish embryonic development are consistent with potentially inhibiting both splice variants. Without being bound by a particular mechanism, the results are consistent with a model in which the interaction of IQ-1 with PR72/130 results in the disruption of the PR72/130 Nkd complex thereby modulating Wnt signaling. The morphological effects on zebrafish development are consistent with a model wherein IQ-1 inhibits the "negative" regulation of canonical Wnt/β-catenin signaling and a concurrent switch to "non-canonical" convergent extension (Fig 5C).

*In vitro* phosphorylation of recombinant p300 by PKC, an enzyme activated via "non-canonical" Wnt signaling, increased the affinity of the β-catenin for p300. Furthermore in cells, IQ-1 significantly decreased Wnt-stimulated phosphorylation of p300 at Ser89, without affecting the overall cellular level of p300. The mechanism by which IQ-1 can decrease the phosphorylation status of p300 at Ser89 remains unclear and is the subject of ongoing investigations.

IQ-1 selectively promoted the β-catenin/CBP interaction at the expense of the corresponding β-catenin/p300 interaction. IQ-1 by enhancing Wnt/β-catenin/CBP-mediated transcription and preventing the switch to Wnt/β-catenin/p300-mediated transcription allows for the long term expansion of murine ESCs while maintaining pluripotency without MEFs or serum.

Differential coactivator usage in Wnt/β-catenin signaling appears to be a critical regulator in the maintenance of the "stem/progenitor" state, the initiation of differentiation with a more restricted proliferative capacity, as well as the switch from "canonical" to "non-canonical" Wnt signaling. IQ-1's effects on Wnt-mediated pluripotency are associated with inhibition of the "negative" regulation of canonical Wnt/β-catenin signaling by the Nkd/PR72/PP2A complex, thereby increasing β-catenin/CBP-driven transcription at the expense of β-catenin/p300-driven transcription.

The ability to expand "stem/progenitor" populations under defined growth conditions has important ramifications in the area of regenerative medicine, and the Examples herein support such use.

### References

1 Willert, K., Brown, J. D., Danenberg, E., Duncan, A. W., Weissman, I. L., Reya, T., Yates, JR 3rd & Nusse, R. (2003) Nature 423, 448-452.
2 Haegele, L., Ingold, B., Naumann, H., Tabatabai, G., Ledermann, B. & Brandner, S. (2003) Mol. Cell Neurosci.24, 696-708.
3 Sato, N., Meijer, L., Skaltsounis, L., Greengard, P. & Brivanlou, A. (2004) Nat. Med. 10, 55-63.
4 Chenn, A. & Walsh, C. A. (2002) Science 297, 365-369.
5 Otero, J. J., Fu, W., Kan, L., Cuadra, A. E. & Kessler, J. A. (2004) Development 131, 3545-3557(2004).
6 Muroyama, Y., Kondoh, H. & Takada, S. (2004) Biochem. Biophys. Res. Commun. 313, 915-921.
7 Teo, J. L., Ma, H., Nguyen, C., Lam, C. & Kahn, M. (2005) Proc. Natl. Acad. Sci. U.S. A. 102, 12171-12176.
8 McMillan, M. & Kahn, M. (2005) Drug Discov. Today 10, 1467-1474.
9 Xu, C., Inokuma, M. S., Denham, J., Golds, K., Kundu, P., Gold, J. D., & Carpenter, M. (2001) Nat. Biotech. 19, 971-974.
10 Lu, J., Hou, R, Booth, C. J., Shih-Hung Yang, S and Snyder, M. (2006) Proc Natl Acad Sci U.S.A. 103, 5688-5693.
11 Yao, S., Chen, S., Clark, J., Hao, E., Beattie, G. M., Hayek, A. & Ding, S. (2006) Proc Natl Acad Sci U.S.A. 103, 6907-6912.
12 Pease, S., Braghetta, P., Gearing, D., Grail, D. & Williams, RL. (1990) Dev. Biol. 141, 344-352.
13 Smith, A. G., Heath,J. K., Donaldson, D. D., Wong, G. G., Moreau, J., Stahl, M. & Rogers, D. (1988) Nature 336, 688-690.
14 Williams, R. L., Hilton, D. J., Pease, S., Willson, T. A., Stewart, C. L., Gearing, D. P., Wagner, E. F., Metcalf, D., Nicola, N. A. & Gough, N. M. (1988) Nature 336,684-687.
15 Niwa, H., Burdon, T., Chambers, I., & Smith, A. (1998) Genes Dev. 12, 2048-2060.
16 Humphrey, R. K., Beattie, G. M., Lopez, A. D., Bucay, N., King, C. C., Firpo, M. T., Rose-John, S. & Hayek, A. (2004) Stem Cells 22, 522-530.
17 Daheron, L., Opitz, S. L., Zaehres, H., Lensch, W. M., Andrews, P. W., Itskovitz-Eldor, J. & Daley, G. Q. (2004) Stem Cells 22, 770-778.
18 Mitsui, K., Tokuzawa, Y., Itoh, H., Segawa, K., Murakami, M., Takahashi, K., Maruyama, M., Maeda,M. & Yamanaka, S. (2003) Cell 113, 631-642.
19 Chambers, I., Colby, D., Robertson, M., Nichols, J., Lee, S., Tweedie, S. & Smith, A. (2003) Cell 113, 643-655.
20 Mumby, M. C. & Waler, G. (1993) Physiol. Rev. 73, 673-699.
21 Hendrix, P., Mayer-Jackel, R E., Cron, P., Goris, J., Hofsteenge, J., Merlevede, W., Hemmings, B. A. (1993) J. Biol. Chem. 268, 15267-15276.
22 Seeling, J. M., Miller, J. R., Gil, R., Moon, R. T., White, R. & Virshup, D. M. (1999) Science 283, 2089-2091.
23 Ratcliffe, M. J., Itoh, K. & Sokol, S. Y. (2000) J. Biol. Chem. 275, 35680-35683.
24 Zeng, W., Wharton, K. A. Jr, Mack, J. A., Wang, K., Gadbaw, M., Suyama, K., Klein, P. S., Scott, M. P. (2000) Nature 403, 789-795.
25 Creyghton, M. P., Roël, G., Eichhorn, P. J. A., Hijmans, E. M., Maurer, I., Destrée, O. & Bernards, R. (2005) Genes. Dev. 19, 376-386.
26 Emami, K. H., Nguyen, C., Ma, H., Kim, D. H., Jeong, K. W., Eguchi, M., Moon, R. T., Teo, J. L., Kim, H. Y., Moon, S. H., et al. (2004) Proc. Natl. Acad. Sci. U. S. A. 101, 12682-12687.
27 Ma, H., Nguyen, C., Lee, K. S. & Kahn, M. (2005) Oncogene 24, 3619-3631.
28 Korinek, V., Barker, N., Morin, P. J., van Wichen, D., de Weger, R., Kinzler, K. W., Vogelstein, B. & Clevers, H. (1997) Science 275, 1784-1787.
29 Kohn, A. D. & Moon, R. T. (2005) Cell Calcium 38, 439-446.
30 Yuan, L. W. & Gambee, J. E. (2000) J. Biol. Chem. 275, 40946-40951.
31 Boyer, L. A., Lee, T.I., Cole, M. F., Johnstone, S. E., Levine, S. S., Zucker, J. P., Guenther, M. G., Kumar, R. M., Murray, H. L., Jenner, R. G. et al. (2005) Cell 122, 947-956.
32 Feuring-Buske, M. & Hogge, D. E. (2001) Blood 97, 3882-3889.
33 Wilson, A., Murphy, M.J., Oskarsson, T., Kaloulis, K., Bettess, M. D., Oser, G. M., Pasche, A. C., Knabenhans, C., Macdonald, H. R. & Trumpp, A. (2004) Genes Dev 18, 2747-2763.
34 Creyghton, M. P., Roel, G., Eichorn, P.J.A., Vredeveld, L.C., Destree, O. & Bernards, R. (2006) Proc. Natl. Acad. Sci. U. S. A. 103, 5397-5402.
35 Zandstra, P. W., Le, H. V., Daley, G. Q., Griffith, L. G. & Lauffenburger, D. A. (2000) Biotechnol Bioeng. 69, 607-617.

## Claims

1. A method of identifying an agent as capable of preventing differentiation while promoting proliferation of a mammalian stem cell during *ex vivo* culture, said method comprising contacting an agent with at least one of subunits PR72/130 of the serine/threonine protein phosphatase PP2A and detecting binding of said agent to said at least one subunit.

2. The method of claim 1, wherein said binding is detected by immunoblotting.

3. The method of claim 1 or claim 2, wherein said agent is selected from the group consisting of compounds of formula 1-5, 7-12, 15 and 17-28.

## Patentansprüche

1. Verfahren zur Identifizierung der Fähigkeit eines Mittels, während einer *ex-vivo-*Kultur die Vermehrung einer Säugetier-Stammzelle zu fördern und dabei die Ausdifferenzierung zu verhindern, wobei das Verfahren das Inkontaktbringen eines Mittels mit mindestens einer der Untereinheiten PR72/130 der Serin/Threonin-Proteinphosphatase PP2A sowie das Detektieren der Bindung des Mittels an die mindestens eine Untereinheit umfasst.

2. Verfahren nach Anspruch 1, wobei die Bindung durch Immunblotting detektiert wird.

3. Verfahren nach Anspruch 1 oder Anspruch 2, wobei das Mittel aus der Gruppe gewählt ist, die aus den Verbindungen der Formeln 1-5, 7-12, 15 und 17-28 besteht.

## Revendications

1. Procédé permettant d'identifier la capacité d'un agent à promouvoir la prolifération d'une cellule souche de mammifère durant une culture *ex vivo* tout en empêchant la différentiation, ledit procédé comprenant la mise en contact d'un agent avec au moins une des sous-unités PR72/130 de la sérine/thréonine protéine phosphatase PP2A ainsi que la détection de l'établissement d'une liaison entre ledit agent et ladite au moins une sous-unité.

2. Procédé selon la revendication 1, ladite liaison étant détectée en réalisant des immunoblots.

3. Procédé selon la revendication 1 ou la revendication 2, ledit agent étant choisi dans le groupe constitué par les composés des formules 1-5, 7-12, 15 et 17-28.


### SEQUENCE LISTING

<110> INSTITUTE FOR CHEMICAL GENOMICS
<120> SERUM-FREE EXPANSION OF CELLS IN CULTURE
<130> 67648-17
<150> 60/740,173
   <151> 2005-11-28
<160> 18
<170> FastSEQ for Windows version 4.0
<210> 1
   <211> 2440
   <212> PRT
   <213> Homo sapiens
<400> 1
   Met Ala Glu Asn Leu Leu Asp Gly Pro Pro Asn Pro Lys Arg Ala Lys
    1               5                   10                  15
   Leu Ser Ser Pro Gly Phe Ser Ala Asn Ser Asn Thr Asp Phe Gly Ser
               20                  25                  30
   Leu Phe Asp Leu Glu Asn Asp Leu Pro Asp Glu Leu Ile Pro Asn Gly
           35                  40                  45
   Gly Glu Leu Gly Leu Leu Asn Ser Gly Asn Leu Val Pro Asp Ala Ala
       50                  55                  60
   Ser Lys His Lys Gln Leu Ser Glu Leu Leu Arg Gly Gly Ser Gly Ser
   65                  70                  75                  80
   Ser Ile Asn Pro Gly Ile Gly Asn Val Ser Ala Ser Ser Pro Val Gln
                   85                  90                  95
   Gln Gly Leu Gly Gly Gln Ala Gln Gly Gln Pro Asn Ser Ala Asn Met
               100                 105                 110
   Ala Ser Leu Ser Ala Met Gly Lys Ser Pro Leu Ser Gln Gly Asp Ser
           115                 120                 125
   Ser Ser Pro Ser Leu Pro Lys Gln Ala Ala Ser Thr Ser Gly Pro Thr
       130                 135                 140
   Pro Ala Ala Ser Gln Ala Leu Asn Pro Gln Ala Gln Lys Gln Val Gly
   145                 150                 155                 160
   Leu Ala Thr Ser Ser Pro Ala Thr Ser Gln Thr Gly Pro Gly Ile Cys
                   165                 170                 175
   Met Asn Ala Asn Phe Asn Gln Thr His Pro Gly Leu Leu Asn Ser Asn
               180                 185                 190
   Ser Gly His Ser Leu Ile Asn Gln Ala Ser Gln Gly Gln Ala Gln Val
           195                 200                 205
   Met Asn Gly Ser Leu Gly Ala Ala Gly Arg Gly Arg Gly Ala Gly Met
       210                 215                 220
   Pro Tyr Thr Ala Pro Ala Met Gln Gly Ala Ser Ser Ser Val Leu Ala
   225                 230                 235                 240
   Glu Thr Leu Thr Gln Val Ser Pro Gln Thr Ala Gly His Ala Gly Leu
                   245                 250                 255
   Asn Thr Ala Gln Ala Gly Gly Met Ala Lys Ile Gly Met Asn Gly Thr
               260                 265                 270
   Thr Ser Pro Phe Gly Gln Pro Phe Ser Gln Ala Gly Gly Gln Pro Met
           275                 280                 285
   Gly Ala Thr Gly Val Asn Pro Gln Leu Ala Ser Lys Gln Ser Met Val
       290                 295                 300
   Asn Ser Leu Pro Thr Phe Pro Thr Asp Ile Lys Asn Thr Ser Val Thr
   305                 310                 315                 320
   Asn Val Pro Asn Met Ser Gln Met Gln Thr Ser Val Gly Ile Val Pro
                   325                 330                 335
   Thr Gln Ala Ile Ala Thr Gly Pro Thr Ala Asp Pro Glu Lys Arg Lys
               340                 345                 350
   Leu Ile Gln Gln Gln Leu Val Leu Leu Leu His Ala His Lys Cys Gln
           355                 360                 365
   Arg Arg Glu Gln Ala Asn Gly Glu Val Arg Ala Cys Ser Leu Pro His
       370                 375                 380
   Cys Arg Thr Met Lys Asn Val Leu Asn His Met Thr His Cys Gln Ala
   385                 390                 395                 400
   Gly Lys Ala Cys Gln Val Ala His Cys Ala Ser Ser Arg Gln Ile Ile
                   405                 410                 415
   Ser His Trp Lys Asn Cys Thr Arg His Asp Cys Pro Val Cys Leu Pro
               420                 425                 430
   Leu Lys Asn Ala Ser Asp Lys Arg Asn Gln Gln Thr Ile Leu Gly Ser
           435                 440                 445
   Pro Ala Ser Gly Ile Gln Asn Thr Ile Gly Ser Val Gly Thr Gly Gln
       450                 455                 460
   Gln Asn Ala Thr Ser Leu Ser Asn Pro Asn Pro Ile Asp Pro Ser Ser
   465                 470                 475                 480
   Met Gln Arg Ala Tyr Ala Ala Leu Gly Leu Pro Tyr Met Asn Gln Pro
                   485                 490                 495
   Gln Thr Gln Leu Gln Pro Gln Val Pro Gly Gln Gln Pro Ala Gln Pro
               500                 505                 510
   Gln Thr His Gln Gln Met Arg Thr Leu Asn Pro Leu Gly Asn Asn Pro
           515                 520                 525
   Met Asn Ile Pro Ala Gly Gly Ile Thr Thr Asp Gln Gln Pro Pro Asn
       530                 535                 540
   Leu Ile Ser Glu Ser Ala Leu Pro Thr Ser Leu Gly Ala Thr Asn Pro
   545                 550                 555                 560
   Leu Met Asn Asp Gly Ser Asn Ser Gly Asn Ile Gly Thr Leu Ser Thr
                   565                 570                 575
   Ile Pro Thr Ala Ala Pro Pro Ser Ser Thr Gly Val Arg Lys Gly Trp
               580                 585                 590
   His Glu His Val Thr Gln Asp Leu Arg Ser His Leu Val His Lys Leu
           595                 600                 605
   Val Gln Ala Ile Phe Pro Thr Pro Asp Pro Ala Ala Leu Lys Asp Arg
       610                 615                 620
   Arg Met Glu Asn Leu Val Ala Tyr Ala Lys Lys Val Glu Gly Asp Met
   625                 630                 635                 640
   Tyr Glu Ser Ala Asn Ser Arg Asp Glu Tyr Tyr His Leu Leu Ala Glu
                   645                 650                 655
   Lys Ile Tyr Lys Ile Gln Lys Glu Leu Glu Glu Lys Arg Arg Ser Arg
               660                 665                 670
   Leu His Lys Gln Gly Ile Leu Gly Asn Gln Pro Ala Leu Pro Ala Pro
           675                 680                 685
   Gly Ala Gln Pro Pro Val Ile Pro Gln Ala Gln Ser Val Arg Pro Pro
       690                 695                 700
   Asn Gly Pro Leu Ser Leu Pro Val Asn Arg Met Gln Val Ser Gln Gly
   705                 710                 715                 720
   Met Asn Ser Phe Asn Pro Met Ser Leu Gly Asn Val Gln Leu Pro Gln
                   725                 730                 735
   Ala Pro Met Gly Pro Arg Ala Ala Ser Pro Met Asn His Ser Val Gln
               740                 745                 750
   Met Asn Ser Met Gly Ser Val Pro Gly Met Ala Ile Ser Pro Ser Arg
           755                 760                 765
   Met Pro Gln Pro Pro Asn Met Met Gly Ala His Thr Asn Asn Met Met
       770                 775                 780
   Ala Gln Ala Pro Ala Gln Ser Gln Phe Leu Pro Gln Asn Gln Phe Pro
   785                 790                 795                 800
   Ser Ser Ser Gly Ala Met Ser Val Gly Met Gly Gln Pro Pro Ala Gln
                   805                 810                 815
   Thr Gly Val Ser Gln Gly Gln Val Pro Gly Ala Ala Leu Pro Asn Pro
               820                 825                 830
   Leu Asn Met Leu Gly Pro Gln Ala Ser Gln Leu Pro Cys Pro Pro Val
           835                 840                 845
   Thr Gln Ser Pro Leu His Pro Thr Pro Pro Pro Ala Ser Thr Ala Ala
       850                 855                 860
   Gly Met Pro Ser Leu Gln His Thr Thr Pro Pro Gly Met Thr Pro Pro
   865                 870                 875                 880
   Gln Pro Ala Ala Pro Thr Gln Pro Ser Thr Pro Val Ser Ser Ser Gly
                   885                 890                 895
   Gln Thr Pro Thr Pro Thr Pro Gly Ser Val Pro Ser Ala Thr Gln Thr
                   900                 905                 910
   Gln Ser Thr Pro Thr Val Gln Ala Ala Ala Gln Ala Gln Val Thr Pro
               915                 920                 925
   Gln Pro Gln Thr Pro Val Gln Pro Pro Ser Val Ala Thr Pro Gln Ser
           930                 935                 940
   Ser Gln Gln Gln Pro Thr Pro Val His Ala Gln Pro Pro Gly Thr Pro
   945                 950                 955                 960
   Leu Ser Gln Ala Ala Ala Ser Ile Asp Asn Arg Val Pro Thr Pro Ser
                   965                 970                 975
   Thr Val Ala Ser Ala Glu Thr Asn Ser Gln Gln Pro Gly Pro Asp Val
               980                 985                 990
   Pro Val Leu Glu Met Lys Thr Glu Thr Gln Ala Glu Asp Thr Glu Pro
           995                 1000                1005
   Asp Pro Gly Glu Ser Lys Gly Glu Pro Arg Ser Glu Met Met Glu Glu
       1010                1015                1020
   Asp Leu Gln Gly Ala Ser Gln Val Lys Glu Glu Thr Asp Ile Ala Glu
   1025                1030                1035                1040
   Gln Lys Ser Glu Pro Met Glu Val Glu Asp Lys Lys Pro Glu Val Lys
                   1045                1050                1055
   Val Glu Val Lys Glu Glu Glu Glu Ser Ser Ser Asn Gly Thr Ala Ser
               1060                1065                1070
   Gln Ser Thr Ser Pro Ser Gln Pro Arg Lys Lys Ile Phe Lys Pro Glu
           1075                1080                1085
   Glu Leu Arg Gln Ala Leu Met Pro Thr Leu Glu Ala Leu Tyr Arg Gln
       1090                1095                1100
   Asp Pro Glu Ser Leu Pro Phe Arg Gln Pro Val Asp Pro Gln Leu Leu
   1105                1110                1115                1120
   Gly Ile Pro Asp Tyr Phe Asp Ile Val Lys Asn Pro Met Asp Leu Ser
                   1125                1130                1135
   Thr Ile Lys Arg Lys Leu Asp Thr Gly Gln Tyr Gln Glu Pro Trp Gln
               1140                1145                1150
   Tyr Val Asp Asp Val Trp Leu Met Phe Asn Asn Ala Trp Leu Tyr Asn
           1155                1160                1165
   Arg Lys Thr Ser Arg Val Tyr Lys Phe Cys Ser Lys Leu Ala Glu Val
       1170                1175                1180
   Phe Glu Gln Glu Ile Asp Pro Val Met Gln Ser Leu Gly Tyr Cys Cys
   1185                1190                1195                1200
   Gly Arg Lys Tyr Glu Phe Ser Pro Gln Thr Leu Cys Cys Tyr Gly Lys
                   1205                1210                1215
   Gln Leu Cys Thr Ile Pro Arg Asp Ala Ala Tyr Tyr Ser Tyr Gln Asn
               1220                1225                1230
   Arg Tyr His Phe Cys Glu Lys Cys Phe Thr Glu Ile Gln Gly Glu Asn
           1235                1240                1245
   Val Thr Leu Gly Asp Asp Pro Ser Gln Pro Gln Thr Thr Ile Ser Lys
       1250                1255                1260
   Asp Gln Phe Glu Lys Lys Lys Asn Asp Thr Leu Asp Pro Glu Pro Phe
   1265                1270                1275                1280
   Val Asp Cys Lys Glu Cys Gly Arg Lys Met His Gln Ile Cys Val Leu
                   1285                1290                1295
   His Tyr Asp Ile Ile Trp Pro Ser Gly Phe Val Cys Asp Asn Cys Leu
               1300                1305                1310
   Lys Lys Thr Gly Arg Pro Arg Lys Glu Asn Lys Phe Ser Ala Lys Arg
           1315                1320                1325
   Leu Gln Thr Thr Arg Leu Gly Asn His Leu Glu Asp Arg Val Asn Lys
       1330                1335                1340
   Phe Leu Arg Arg Gln Asn His Pro Glu Ala Gly Glu Val Phe Val Arg
   1345                1350                1355                1360
   Val Val Ala Ser Ser Asp Lys Thr Val Glu Val Lys Pro Gly Met Lys
                   1365                1370                1375
   Ser Arg Phe Val Asp Ser Gly Glu Met Ser Glu Ser Phe Pro Tyr Arg
               1380                1385                1390
   Thr Lys Ala Leu Phe Ala Phe Glu Glu Ile Asp Gly Val Asp Val Cys
           1395                1400                1405
   Phe Phe Gly Met His Val Gln Asp Thr Ala Leu Ile Ala Pro His Gln
       1410                1415                1420
   Ile Gln Gly Cys Val Tyr Ile Ser Tyr Leu Asp Ser Ile His Phe Phe
   1425                1430                1435                1440
   Arg Pro Arg Cys Leu Arg Thr Ala Val Tyr His Glu Ile Leu Ile Gly
                   1445                1450                1455
   Tyr Leu Glu Tyr Val Lys Lys Leu Val Tyr Val Thr Ala His Ile Trp
               1460                1465                1470
   Ala Cys Pro Pro Ser Glu Gly Asp Asp Tyr Ile Phe His Cys His Pro
           1475                1480                1485
   Pro Asp Gln Lys Ile Pro Lys Pro Lys Arg Leu Gln Glu Trp Tyr Lys
       1490                1495                1500
   Lys Met Leu Asp Lys Ala Phe Ala Glu Arg Ile Ile Asn Asp Tyr Lys
   1505                1510                1515                1520
   Asp Ile Phe Lys Gln Ala Asn Glu Asp Arg Leu Thr Ser Ala Lys Glu
                   1525                1530                1535
   Leu Pro Tyr Phe Glu Gly Asp Phe Trp Pro Asn Val Leu Glu Glu Ser
               1540                1545                1550
   Ile Lys Glu Leu Glu Gln Glu Glu Glu Glu Arg Lys Lys Glu Glu Ser
           1555                1560                1565
   Thr Ala Ala Ser Glu Thr Pro Glu Gly Ser Gln Gly Asp Ser Lys Asn
       1570                1575                1580
   Ala Lys Lys Lys Asn Asn Lys Lys Thr Asn Lys Asn Lys Ser Ser Ile
   1585                1590                1595                1600
   Ser Arg Ala Asn Lys Lys Lys Pro Ser Met Pro Asn Val Ser Asn Asp
                   1605                1610                1615
   Leu Ser Gln Lys Leu Tyr Ala Thr Met Glu Lys His Lys Glu Val Phe
               1620                1625                1630
   Phe Val Ile His Leu His Ala Gly Pro Val Ile Ser Thr Gln Pro Pro
           1635                1640                1645
   Ile Val Asp Pro Asp Pro Leu Leu Ser Cys Asp Leu Met Asp Gly Arg
       1650                1655                1660
   Asp Ala Phe Leu Thr Leu Ala Arg Asp Lys His Trp Glu Phe Ser Ser
   1665                1670                1675                1680
   Leu Arg Arg Ser Lys Trp Ser Thr Leu Cys Met Leu Val Glu Leu His
                   1685                1690                1695
   Thr Gln Gly Gln Asp Arg Phe Val Tyr Thr Cys Asn Glu Cys Lys His
               1700                1705                1710
   His Val Glu Thr Arg Trp His Cys Thr Val Cys Glu Asp Tyr Asp Leu
           1715                1720                1725
   Cys Ile Asn Cys Tyr Asn Thr Lys Ser His Thr His Lys Met Val Lys
       1730                1735                1740
   Trp Gly Leu Gly Leu Asp Asp Glu Gly Ser Ser Gln Gly Glu Pro Gln
   1745                1750                1755                1760
   Ser Lys Ser Pro Gln Glu Ser Arg Arg Leu Ser Ile Gln Arg Cys Ile
                   1765                1770                1775
   Gln Ser Leu Val His Ala Cys Gln Cys Arg Asn Ala Asn Cys Ser Leu
               1780                1785                1790
   Pro Ser Cys Gln Lys Met Lys Arg Val Val Gln His Thr Lys Gly Cys
           1795                1800                1805
   Lys Arg Lys Thr Asn Gly Gly Cys Pro Val Cys Lys Gln Leu Ile Ala
       1810                1815                1820
   Leu Cys Cys Tyr His Ala Lys His Cys Gln Glu Asn Lys Cys Pro Val
   1825                1830                1835                1840
   Pro Phe Cys Leu Asn Ile Lys His Asn Val Arg Gln Gln Gln Ile Gln
                   1845                1850                1855
   His Cys Leu Gln Gln Ala Gln Leu Met Arg Arg Arg Met Ala Thr Met
               1860                1865                1870
   Asn Thr Arg Asn Val Pro Gln Gln Ser Leu Pro Ser Pro Thr Ser Ala
           1875                1880                1885
   Pro Pro Gly Thr Pro Thr Gln Gln Pro Ser Thr Pro Gln Thr Pro Gln
       1890                1895                1900
   Pro Pro Ala Gln Pro Gln Pro Ser Pro Val Asn Met Ser Pro Ala Gly
   1905                1910                1915                1920
   Phe Pro Asn Val Ala Arg Thr Gln Pro Pro Thr Ile Val Ser Ala Gly
                   1925                1930                1935
   Lys Pro Thr Asn Gln Val Pro Ala Pro Pro Pro Pro Ala Gln Pro Pro
               1940                1945                1950
   Pro Ala Ala Val Glu Ala Ala Arg Gln Ile Glu Arg Glu Ala Gln Gln
           1955                1960                1965
   Gln Gln His Leu Tyr Arg Ala Asn Ile Asn Asn Gly Met Pro Pro Gly
       1970                1975                1980
   Arg Asp Gly Met Gly Thr Pro Gly Ser Gln Met Thr Pro Val Gly Leu
   1985                1990                1995                2000
   Asn Val Pro Arg Pro Asn Gln Val Ser Gly Pro Val Met Ser Ser Met
                   2005                2010                2015
   Pro Pro Gly Gln Trp Gln Gln Ala Pro Ile Pro Gln Gln Gln Pro Met
               2020                2025                2030
   Pro Gly Met Pro Arg Pro Val Met Ser Met Gln Ala Gln Ala Ala Val
           2035                2040                2045
   Ala Gly Pro Arg Met Pro Asn Val Gln Pro Asn Arg Ser Ile Ser Pro
       2050                2055                2060
   Ser Ala Leu Gln Asp Leu Leu Arg Thr Leu Lys Ser Pro Ser Ser Pro
   2065                2070                2075                2080
   Gln Gln Gln Gln Gln Val Leu Asn Ile Leu Lys Ser Asn Pro Gln Leu
                   2085                2090                2095
   Met Ala Ala Phe Ile Lys Gln Arg Thr Ala Lys Tyr Val Ala Asn Gln
               2100                2105                2110
   Pro Gly Met Gln Pro Gln Pro Gly Leu Gln Ser Gln Pro Gly Met Gln
           2115                2120                2125
   Pro Gln Pro Gly Met His Gln Gln Pro Ser Leu Gln Asn Leu Asn Ala
       2130                2135                2140
   Met Gln Ala Gly Val Pro Arg Pro Gly Val Pro Pro Pro Gln Pro Ala
   2145                2150                2155                2160
   Met Gly Gly Leu Asn Pro Gln Gly Gln Ala Leu Asn Ile Met Asn Pro
                   2165                2170                2175
   Gly His Asn Pro Asn Met Thr Asn Met Asn Pro Gln Tyr Arg Glu Met
               2180                2185                2190
   Val Arg Arg Gln Leu Leu Gln His Gln Gln Gln Gln Gln Gln Gln Gln
           2195                2200                2205
   Gln Gln Gln Gln Gln Gln Gln Asn Ser Ala Ser Leu Ala Gly Gly Met
       2210                2215                2220
   Ala Gly His Ser Gln Phe Gln Gln Pro Gln Gly Pro Gly Gly Tyr Ala
   2225                2230                2235                2240
   Pro Ala Met Gln Gln Gln Arg Met Gln Gln His Leu Pro Ile Gln Gly
                   2245                2250                2255
   Ser Ser Met Gly Gln Met Ala Ala Pro Met Gly Gln Leu Gly Gln Met
               2260                2265                2270
   Gly Gln Pro Gly Leu Gly Ala Asp Ser Thr Pro Asn Ile Gln Gln Ala
           2275                2280                2285
   Leu Gln Gln Arg Ile Leu Gln Gln Gln Gln Met Lys Gln Gln Ile Gly
       2290                2295                2300
   Ser Pro Gly Gln Pro Asn Pro Met Ser Pro Gln Gln His Met Leu Ser
   2305                2310                2315                2320
   Gly Gln Pro Gln Ala Ser His Leu Pro Gly Gln Gln Ile Ala Thr Ser
                   2325                2330                2335
   Leu Ser Asn Gln Val Arg Ser Pro Ala Pro Val Gln Ser Pro Arg Pro
               2340                2345                2350
   Gln Ser Gln Pro Pro His Ser Ser Pro Ser Pro Arg Ile Gln Pro Gln
           2355                2360                2365
   Pro Ser Pro His His Val Ser Pro Gln Thr Gly Thr Pro His Pro Gly
       2370                2375                2380
   Leu Ala Val Thr Met Ala Ser Ser Met Asp Gln Gly His Leu Gly Asn
   2385                2390                2395                2400
   Pro Glu Gln Ser Ala Met Leu Pro Gln Leu Asn Thr Pro Asn Arg Ser
                   2405                2410                2415
   Ala Leu Ser Ser Glu Leu Ser Leu Val Gly Asp Thr Thr Gly Asp Thr
               2420                2425                2430
   Leu Glu Lys Phe Val Glu Gly Leu
           2435                2440
<210> 2
   <211> 2414
   <212> PRT
   <213> Homo sapiens
<400> 2
   Met Ala Glu Asn Val Val Glu Pro Gly Pro Pro Ser Ala Lys Arg Pro
   1               5                   10                  15
   Lys Leu Ser Ser Pro Ala Leu Ser Ala Ser Ala Ser Asp Gly Thr Asp
   20 25 30
   Phe Gly Ser Leu Phe Asp Leu Glu His Asp Leu Pro Asp Glu Leu Ile
   35 40 45
   Asn Ser Thr Glu Leu Gly Leu Thr Asn Gly Gly Asp Ile Asn Gln Leu
   50 55 60
   Gln Thr Ser Leu Gly Met Val Gln Asp Ala Ala Ser Lys His Lys Gln
   65 70 75 80
   Leu Ser Glu Leu Leu Arg Ser Gly Ser Ser Pro Asn Leu Asn Met Gly
   85 90 95
   Val Gly Gly Pro Gly Gln Val Met Ala Ser Gln Ala Gln Gln Ser Ser
   100 105 110
   Pro Gly Leu Gly Leu Ile Asn Ser Met Val Lys Ser Pro Met Thr Gln
   115 120 125
   Ala Gly Leu Thr Ser Pro Asn Met Gly Met Gly Thr Ser Gly Pro Asn
   130 135 140
   Gln Gly Pro Thr Gln Ser Thr Gly Met Met Asn Ser Pro Val Asn Gln
   145 150 155 160
   Pro Ala Met Gly Met Asn Thr Gly Thr Asn Ala Gly Met Asn Pro Gly
   165 170 175
   Met Leu Ala Ala Gly Asn Gly Gln Gly Ile Met Pro Asn Gln Val Met
   180 185 190
   Asn Gly Ser Ile Gly Ala Gly Arg Gly Arg Gln Asp Met Gln Tyr Pro
   195 200 205
   Asn Pro Gly Met Gly Ser Ala Gly Asn Leu Leu Thr Glu Pro Leu Gln
   210 215 220
   Gln Gly Ser Pro Gln Met Gly Gly Gln Thr Gly Leu Arg Gly Pro Gln
   225 230 235 240
   Pro Leu Lys Met Gly Met Met Asn Asn Pro Asn Pro Tyr Gly Ser Pro
   245 250 255
   Tyr Thr Gln Asn Pro Gly Gln Gln Ile Gly Ala Ser Gly Leu Gly Leu
   260 265 270
   Gln Ile Gln Thr Lys Thr Val Leu Ser Asn Asn Leu Ser Pro Phe Ala
   275 280 285
   Met Asp Lys Lys Ala Val Pro Gly Gly Gly Met Pro Asn Met Gly Gln
   290 295 300
   Gln Pro Ala Pro Gln Val Gln Gln Pro Gly Leu Val Thr Pro Val Ala
   305 310 315 320
   Gln Gly Met Gly Ser Gly Ala His Thr Ala Asp Pro Glu Lys Arg Lys
   325 330 335
   Leu Ile Gln Gln Gln Leu Val Leu Leu Leu His Ala His Lys Cys Gln
   340 345 350
   Arg Arg Glu Gln Ala Asn Gly Glu Val Arg Gln Cys Asn Leu Pro His
   355 360 365
   Cys Arg Thr Met Lys Asn Val Leu Asn His Met Thr His Cys Gln Ser
   370 375 380
   Gly Lys Ser Cys Gln Val Ala His Cys Ala Ser Ser Arg Gln Ile Ile
   385 390 395 400
   Ser His Trp Lys Asn Cys Thr Arg His Asp Cys Pro Val Cys Leu Pro
   405 410 415
   Leu Lys Asn Ala Gly Asp Lys Arg Asn Gln Gln Pro Ile Leu Thr Gly
   420 425 430
   Ala Pro Val Gly Leu Gly Asn Pro Ser Ser Leu Gly Val Gly Gln Gln
   435 440 445
   Ser Ala Pro Asn Leu Ser Thr Val Ser Gln Ile Asp Pro Ser Ser Ile
   450 455 460
   Glu Arg Ala Tyr Ala Ala Leu Gly Leu Pro Tyr Gln Val Asn Gln Met
   465 470 475 480
   Pro Thr Gln Pro Gln Val Gln Ala Lys Asn Gln Gln Asn Gln Gln Pro
   485 490 495
   Gly Gln Ser Pro Gln Gly Met Arg Pro Met Ser Asn Met Ser Ala Ser
   500 505 510
   Pro Met Gly Val Asn Gly Gly Val Gly Val Gln Thr Pro Ser Leu Leu
   515 520 525
   Ser Asp Ser Met Leu His Ser Ala Ile Asn Ser Gln Asn Pro Met Met
   530 535 540
   Ser Glu Asn Ala Ser Val Pro Ser Leu Gly Pro Met Pro Thr Ala Ala
   545 550 555 560
   Gln Pro Ser Thr Thr Gly Ile Arg Lys Gln Trp His Glu Asp Ile Thr
                       565                     570                     575
   Gln Asp Leu Arg Asn His Leu Val His Lys Leu Val Gln Ala Ile Phe
                   580                     585                     590
   Pro Thr Pro Asp Pro Ala Ala Leu Lys Asp Arg Arg Met Glu Asn Leu
               595                     600                     605
   Val Ala Tyr Ala Arg Lys Val Glu Gly Asp Met Tyr Glu Ser Ala Asn
       610                     615                     620
   Asn Arg Ala Glu Tyr Tyr His Leu Leu Ala Glu Lys Ile Tyr Lys Ile
   625                     630                     635                     640
   Gln Lys Glu Leu Glu Glu Lys Arg Arg Thr Arg Leu Gln Lys Gln Asn
                   645                     650                     655
   Met Leu Pro Asn Ala Ala Gly Met Val Pro Val Ser Met Asn Pro Gly
               660                     665                     670
   Pro Asn Met Gly Gln Pro Gln Pro Gly Met Thr Ser Asn Gly Pro Leu
           675                     680                     685
   Pro Asp Pro Ser Met Ile Arg Gly Ser Val Pro Asn Gln Met Met Pro
       690                     695                     700
   Arg Ile Thr Pro Gln Ser Gly Leu Asn Gln Phe Gly Gln Met Ser Met
   705                     710                     715                     720
   Ala Gln Pro Pro Ile Val Pro Arg Gln Thr Pro Pro Leu Gln His His
                   725                     730                     735
   Gly Gln Leu Ala Gln Pro Gly Ala Leu Asn Pro Pro Met Gly Tyr Gly
               740                     745                     750
   Pro Arg Met Gln Gln Pro Ser Asn Gln Gly Gln Phe Leu Pro Gln Thr
           755                     760                     765
   Gln Phe Pro Ser Gln Gly Met Asn Val Thr Asn Ile Pro Leu Ala Pro
       770                     775                     780
   Ser Ser Gly Gln Ala Pro Val Ser Gln Ala Gln Met Ser Ser Ser Ser
   785                     790                     795                     800
   Cys Pro Val Asn Ser Pro Ile Met Pro Pro Gly Ser Gln Gly Ser His
                   805                     810                     815
   Ile His Cys Pro Gln Leu Pro Gln Pro Ala Leu His Gln Asn Ser Pro
               820                     825                     830
   Ser Pro Val Pro Ser Arg Thr Pro Thr Pro His His Thr Pro Pro Ser
           835                     840                     845
   Ile Gly Ala Gln Gln Pro Pro Ala Thr Thr Ile Pro Ala Pro Val Pro
       850                     855                     860
   Thr Pro Pro Ala Met Pro Pro Gly Pro Gln Ser Gln Ala Leu His Pro
   865                     870                     875                     880
   Pro Pro Arg Gln Thr Pro Thr Pro Pro Thr Thr Gln Leu Pro Gln Gln
                   885                     890                     895
   Val Gln Pro Ser Leu Pro Ala Ala Pro Ser Ala Asp Gln Pro Gln Gln
               900                     905                     910
   Gln Pro Arg Ser Gln Gln Ser Thr Ala Ala Ser Val Pro Thr Pro Asn
           915                     920                     925
   Ala Pro Leu Leu Pro Pro Gln Pro Ala Thr Pro Leu Ser Gln Pro Ala
       930                     935                     940
   Val Ser Ile Glu Gly Gln Val Ser Asn Pro Pro Ser Thr Ser Ser Thr
   945                     950                     955                     960
   Glu Val Asn Ser Gln Ala Ile Ala Glu Lys Gln Pro Ser Gln Glu Val
                   965                     970                     975
   Lys Met Glu Ala Lys Met Glu Val Asp Gln Pro Glu Pro Ala Asp Thr
               980                     985                     990
   Gln Pro Glu Asp Ile Ser Glu Ser Lys Val Glu Asp Cys Lys Met Glu
           995                     1000                    1005
   Ser Thr Glu Thr Glu Glu Arg Ser Thr Glu Leu Lys Thr Glu Ile Lys
       1010                    1015                    1020
   Glu Glu Glu Asp Gln Pro Ser Thr Ser Ala Thr Gln Ser Ser Pro Ala
   1025                    1030                    1035                    1040
   Pro Gly Gln Ser Lys Lys Lys Ile Phe Lys Pro Glu Glu Leu Arg Gln
                   1045                    1050                    1055
   Ala Leu Met Pro Thr Leu Glu Ala Leu Tyr Arg Gln Asp Pro Glu Ser
               1060                    1065                    1070
   Leu Pro Phe Arg Gln Pro Val Asp Pro Gln Leu Leu Gly Ile Pro Asp
           1075                    1080                    1085
   Tyr Phe Asp Ile Val Lys Ser Pro Met Asp Leu Ser Thr Ile Lys Arg
       1090                    1095                    1100
   Lys Leu Asp Thr Gly Gln Tyr Gln Glu Pro Trp Gln Tyr Val Asp Asp
   1105                1110                1115                1120
   Ile Trp Leu Met Phe Asn Asn Ala Trp Leu Tyr Asn Arg Lys Thr Ser
                   1125                1130                1135
   Arg Val Tyr Lys Tyr Cys Ser Lys Leu Ser Glu Val Phe Glu Gln Glu
               1140                1145                1150
   Ile Asp Pro Val Met Gln Ser Leu Gly Tyr Cys Cys Gly Arg Lys Leu
           1155                1160                1165
   Glu Phe Ser Pro Gln Thr Leu Cys Cys Tyr Gly Lys Gln Leu Cys Thr
       1170                1175                1180
   Ile Pro Arg Asp Ala Thr Tyr Tyr Ser Tyr Gln Asn Arg Tyr His Phe
   1185                1190                1195                1200
   Cys Glu Lys Cys Phe Asn Glu Ile Gln Gly Glu Ser Val Ser Leu Gly
                   1205                1210                1215
   Asp Asp Pro Ser Gln Pro Gln Thr Thr Ile Asn Lys Glu Gln Phe Ser
               1220                1225                1230
   Lys Arg Lys Asn Asp Thr Leu Asp Pro Glu Leu Phe Val Glu Cys Thr
           1235                1240                1245
   Glu Cys Gly Arg Lys Met His Gln Ile Cys Val Leu His His Glu Ile
       1250                1255                1260
   Ile Trp Pro Ala Gly Phe Val Cys Asp Gly Cys Leu Lys Lys Ser Ala
   1265                1270                1275                1280
   Arg Thr Arg Lys Glu Asn Lys Phe Ser Ala Lys Arg Leu Pro Ser Thr
                   1285                1290                1295
   Arg Leu Gly Thr Phe Leu Glu Asn Arg Val Asn Asp Phe Leu Arg Arg
               1300                1305                1310
   Gln Asn His Pro Glu Ser Gly Glu Val Thr Val Arg Val Val His Ala
           1315                1320                1325
   Ser Asp Lys Thr Val Glu Val Lys Pro Gly Met Lys Ala Arg Phe Val
       1330                1335                1340
   Asp Ser Gly Glu Met Ala Glu Ser Phe Pro Tyr Arg Thr Lys Ala Leu
   1345                1350                1355                1360
   Phe Ala Phe Glu Glu Ile Asp Gly Val Asp Leu Cys Phe Phe Gly Met
                   1365                1370                1375
   His Val Gln Glu Tyr Gly Ser Asp Cys Pro Pro Pro Asn Gln Arg Arg
               1380                1385                1390
   Val Tyr Ile Ser Tyr Leu Asp Ser Val His Phe Phe Arg Pro Lys Cys
           1395                1400                1405
   Leu Arg Thr Ala Val Tyr His Glu Ile Leu Ile Gly Tyr Leu Glu Tyr
       1410                1415                1420
   Val Lys Lys Leu Gly Tyr Thr Thr Gly His Ile Trp Ala Cys Pro Pro
   1425                1430                1435                1440
   Ser Glu Gly Asp Asp Tyr Ile Phe His Cys His Pro Pro Asp Gln Lys
                   1445                1450                1455
   Ile Pro Lys Pro Lys Arg Leu Gln Glu Trp Tyr Lys Lys Met Leu Asp
               1460                1465                1470
   Lys Ala Val Ser Glu Arg Ile Val His Asp Tyr Lys Asp Ile Phe Lys
           1475                1480                1485
   Gln Ala Thr Glu Asp Arg Leu Thr Ser Ala Lys Glu Leu Pro Tyr Phe
       1490                1495                1500
   Glu Gly Asp Phe Trp Pro Asn Val Leu Glu Glu Ser Ile Lys Glu Leu
   1505                1510                1515                1520
   Glu Gln Glu Glu Glu Glu Arg Lys Arg Glu Glu Asn Thr Ser Asn Glu
                   1525                1530                1535
   Ser Thr Asp Val Thr Lys Gly Asp Ser Lys Asn Ala Lys Lys Lys Asn
               1540                1545                1550
   Asn Lys Lys Thr Ser Lys Asn Lys Ser Ser Leu Ser Arg Gly Asn Lys
           1555                1560                1565
   Lys Lys Pro Gly Met Pro Asn Val Ser Asn Asp Leu Ser Gln Lys Leu
       1570                1575                1580
   Tyr Ala Thr Met Glu Lys His Lys Glu Val Phe Phe Val Ile Arg Leu
   1585                1590                1595                1600
   Ile Ala Gly Pro Ala Ala Asn Ser Leu Pro Pro Ile Val Asp Pro Asp
                   1605                1610                1615
   Pro Leu Ile Pro Cys Asp Leu Met Asp Gly Arg Asp Ala Phe Leu Thr
               1620                1625                1630
   Leu Ala Arg Asp Lys His Leu Glu Phe Ser Ser Leu Arg Arg Ala Gln
           1635                1640                1645
   Trp Ser Thr Met Cys Met Leu Val Glu Leu His Thr Gln Ser Gln Asp
       1650                1655                1660
   Arg Phe Val Tyr Thr Cys Asn Glu Cys Lys His His Val Glu Thr Arg
   1665                1670                1675                1680
   Trp His Cys Thr Val Cys Glu Asp Tyr Asp Leu Cys Ile Thr Cys Tyr
                   1685                1690                1695
   Asn Thr Lys Asn His Asp His Lys Met Glu Lys Leu Gly Leu Gly Leu
               1700                1705                1710
   Asp Asp Glu Ser Asn Asn Gln Gln Ala Ala Ala Thr Gln Ser Pro Gly
           1715                1720                1725
   Asp Ser Arg Arg Leu Ser Ile Gln Arg Cys Ile Gln Ser Leu Val His
       1730                1735                1740
   Ala Cys Gln Cys Arg Asn Ala Asn Cys Ser Leu Pro Ser Cys Gln Lys
   1745                1750                1755                1760
   Met Lys Arg Val Val Gln His Thr Lys Gly Cys Lys Arg Lys Thr Asn
                   1765                1770                1775
   Gly Gly Cys Pro Ile Cys Lys Gln Leu Ile Ala Leu Cys Cys Tyr His
               1780                1785                1790
   Ala Lys His Cys Gln Glu Asn Lys Cys Pro Val Pro Phe Cys Leu Asn
           1795                1800                1805
   Ile Lys Gln Lys Leu Arg Gln Gln Gln Leu Gln His Arg Leu Gln Gln
       1810                1815                1820
   Ala Gln Met Leu Arg Arg Arg Met Ala Ser Met Gln Arg Thr Gly Val
   1825                1830                1835                1840
   Val Gly Gln Gln Gln Gly Leu Pro Ser Pro Thr Pro Ala Thr Pro Thr
                   1845                1850                1855
   Thr Pro Thr Gly Gln Gln Pro Thr Thr Pro Gln Thr Pro Gln Pro Thr
               1860                1865                1870
   Ser Gln Pro Gln Pro Thr Pro Pro Asn Ser Met Pro Pro Tyr Leu Pro
           1875                1880                1885
   Arg Thr Gln Ala Ala Gly Pro Val Ser Gln Gly Lys Ala Ala Gly Gln
       1890                1895                1900
   Val Thr Pro Pro Thr Pro Pro Gln Thr Ala Gln Pro Pro Leu Pro Gly
   1905                1910                1915                1920
   Pro Pro Pro Thr Ala Val Glu Met Ala Met Gln Ile Gln Arg Ala Ala
                   1925                1930                1935
   Glu Thr Gln Arg Gln Met Ala His Val Gln Ile Phe Gln Arg Pro Ile
               1940                1945                1950
   Gln His Gln Met Pro Pro Met Thr Pro Met Ala Pro Met Gly Met Asn
           1955                1960                1965
   Pro Pro Pro Met Thr Arg Gly Pro Ser Gly His Leu Glu Pro Gly Met
       1970                1975                1980
   Gly Pro Thr Gly Met Gln Gln Gln Pro Pro Trp Ser Gln Gly Gly Leu
   1985                1990                1995                2000
   Pro Gln Pro Gln Gln Leu Gln Ser Gly Met Pro Arg Pro Ala Met Met
                   2005                2010                2015
   Ser Val Ala Gln His Gly Gln Pro Leu Asn Met Ala Pro Gln Pro Gly
               2020                2025                2030
   Leu Gly Gln Val Gly Ile Ser Pro Leu Lys Pro Gly Thr Val Ser Gln
           2035                2040                2045
   Gln Ala Leu Gln Asn Leu Leu Arg Thr Leu Arg Ser Pro Ser Ser Pro
       2050                2055                2060
   Leu Gln Gln Gln Gln Val Leu Ser Ile Leu His Ala Asn Pro Gln Leu
   2065                2070                2075                2080
   Leu Ala Ala Phe Ile Lys Gln Arg Ala Ala Lys Tyr Ala Asn Ser Asn
                   2085                2090                2095
   Pro Gln Pro Ile Pro Gly Gln Pro Gly Met Pro Gln Gly Gln Pro Gly
               2100                2105                2110
   Leu Gln Pro Pro Thr Met Pro Gly Gln Gln Gly Val His Ser Asn Pro
           2115                2120                2125
   Ala Met Gln Asn Met Asn Pro Met Gln Ala Gly Val Gln Arg Ala Gly
       2130                2135                2140
   Leu Pro Gln Gln Gln Pro Gln Gln Gln Leu Gln Pro Pro Met Gly Gly
   2145                2150                2155                2160
   Met Ser Pro Gln Ala Gln Gln Met Asn Met Asn His Asn Thr Met Pro
                   2165                2170                2175
   Ser Gln Phe Arg Asp Ile Leu Arg Arg Gln Gln Met Met Gln Gln Gln
               2180                2185                2190
   Gln Gln Gln Gly Ala Gly Pro Gly Ile Gly Pro Gly Met Ala Asn His
               2195                    2200                    2205
   Asn Gln Phe Gln Gln Pro Gln Gly Val Gly Tyr Pro Pro Gln Pro Gln
           2210                    2215                    2220
   Gln Arg Met Gln His His Met Gln Gln Met Gln Gln Gly Asn Met Gly
   2225                    2230                    2235                    2240
   Gln Ile Gly Gln Leu Pro Gln Ala Leu Gly Ala Glu Ala Gly Ala Ser
                   2245                    2250                    2255
   Leu Gln Ala Tyr Gln Gln Arg Leu Leu Gln Gln Gln Met Gly Ser Pro
               2260                    2265                    2270
   Val Gln Pro Asn Pro Met Ser Pro Gln Gln His Met Leu Pro Asn Gln
           2275                    2280                    2285
   Ala Gln Ser Pro His Leu Gln Gly Gln Gln Ile Pro Asn Ser Leu Ser
       2290                    2295                    2300
   Asn Gln Val Arg Ser Pro Gln Pro Val Pro Ser Pro Arg Pro Gln Ser
   2305                    2310                    2315                    2320
   Gln Pro Pro His Ser Ser Pro Ser Pro Arg Met Gln Pro Gln Pro Ser
                   2325                    2330                    2335
   Pro His His Val Ser Pro Gln Thr Ser Ser Pro His Pro Gly Leu Val
               2340                    2345                    2350
   Ala Ala Gln Ala Asn Pro Met Glu Gln Gly His Phe Ala Ser Pro Asp
           2355                    2360                    2365
   Gln Asn Ser Met Leu Ser Gln Leu Ala Ser Asn Pro Gly Met Ala Asn
       2370                    2375                    2380
   Leu His Gly Ala Ser Ala Thr Asp Leu Gly Leu Ser Thr Asp Asn Ser
   2385                    2390                    2395                    2400
   Asp Leu Asn Ser Asn Leu Ser Gln Ser Thr Leu Asp Ile His
                   2405                    2410
<210> 3
   <211> 781
   <212> PRT
   <213> Homo sapiens
<400> 3
<210> 4
   <211> 745
   <212> PRT
   <213> Homo sapiens
<400> 4
   Met Glu Val Met Asn Leu Met Glu Gln Pro Ile Lys Val Thr Glu Trp
    1              5                   10                  15
   Gln Gln Thr Tyr Thr Tyr Asp Ser Gly Ile His Ser Gly Ala Asn Thr
               20                  25                  30
   Cys Val Pro Ser Val Ser Ser Lys Gly Ile Met Glu Glu Asp Glu Ala
           35                  40                  45
   Cys Gly Arg Gln Tyr Thr Leu Lys Lys Thr Thr Thr Tyr Thr Gln Gly
       50                  55                  60
   Val Pro Pro Ser Gln Gly Asp Leu Glu Tyr Gln Met Ser Thr Thr Ala
   65                  70                  75                  80
   Arg Ala Lys Arg Val Arg Glu Ala Met Cys Pro Gly Val Ser Gly Glu
                   85                  90                  95
   Asp Ser Ser Leu Leu Leu Ala Thr Gln Val Glu Gly Gln Ala Thr Asn
               100                 105                 110
   Leu Gln Arg Leu Ala Glu Pro Ser Gln Leu Leu Lys Ser Ala Ile Val
           115                 120                 125
   His Leu Ile Asn Tyr Gln Asp Asp Ala Glu Leu Ala Thr Arg Ala Leu
       130                 135                 140
   Pro Glu Leu Thr Lys Leu Leu Asn Asp Glu Asp Pro Val Val Val Thr
   145                 150                 155                 160
   Lys Ala Ala Met Ile Val Asn Gln Leu Ser Lys Lys Glu Ala Ser Arg
                   165                 170                 175
   Arg Ala Leu Met Gly Ser Pro Gln Leu Val Ala Ala Val Val Arg Thr
               180                 185                 190
   Met Gln Asn Thr Ser Asp Leu Asp Thr Ala Arg Cys Thr Thr Ser Ile
           195                 200                 205
   Leu His Asn Leu Ser His His Arg Glu Gly Leu Leu Ala Ile Phe Lys
       210                 215                 220
   Ser Gly Gly Ile Pro Ala Leu Val Arg Met Leu Ser Ser Pro Val Glu
   225                 230                 235                 240
   Ser Val Leu Phe Tyr Ala Ile Thr Thr Leu His Asn Leu Leu Leu Tyr
                   245                 250                 255
   Gln Glu Gly Ala Lys Met Ala Val Arg Leu Ala Asp Gly Leu Gln Lys
               260                 265                 270
   Met Val Pro Leu Leu Asn Lys Asn Asn Pro Lys Phe Leu Ala Ile Thr
           275                 280                 285
   Thr Asp Cys Leu Gln Leu Leu Ala Tyr Gly Asn Gln Glu Ser Lys Leu
       290                 295                 300
   Ile Ile Leu Ala Asn Gly Gly Pro Gln Ala Leu Val Gln Ile Met Arg
   305                 310                 315                 320
   Asn Tyr Ser Tyr Glu Lys Leu Leu Trp Thr Thr Ser Arg Val Leu Lys
                   325                 330                 335
   Val Leu Ser Val Cys Pro Ser Asn Lys Pro Ala Ile Val Glu Ala Gly
               340                 345                 350
   Gly Met Gln Ala Leu Gly Lys His Leu Thr Ser Asn Ser Pro Arg Leu
           355                 360                 365
   Val Gln Asn Cys Leu Trp Thr Leu Arg Asn Leu Ser Asp Val Ala Thr
       370                 375                 380
   Lys Gln Glu Gly Leu Glu Ser Val Leu Lys Ile Leu Val Asn Gln Leu
   385                 390                 395                 400
   Ser Val Asp Asp Val Asn Val Leu Thr Cys Ala Thr Gly Thr Leu Ser
                   405                 410                 415
   Asn Leu Thr Cys Asn Asn Ser Lys Asn Lys Thr Leu Val Thr Gln Asn
               420                 425                 430
   Ser Gly Val Glu Ala Leu Ile His Ala Ile Leu Arg Ala Gly Asp Lys
           435                 440                 445
   Asp Asp Ile Thr Glu Pro Ala Val Cys Ala Leu Arg His Leu Thr Ser
       450                 455                 460
   Arg His Pro Glu Ala Glu Met Ala Gln Asn Ser Val Arg Leu Asn Tyr
   465                 470                 475                 480
   Gly Ile Pro Ala Ile Val Lys Leu Leu Asn Gln Pro Asn Gln Trp Pro
                   485                 490                 495
   Leu Val Lys Ala Thr Ile Gly Leu Ile Arg Asn Leu Ala Leu Cys Pro
                       500                     505                     510
   Ala Asn His Ala Pro Leu Gln Glu Ala Ala Val Ile Pro Arg Leu Val
               515                     520                     525
   Gln Leu Leu Val Lys Ala His Gln Asp Ala Gln Arg His Val Ala Ala
           530                     535                     540
   Gly Thr Gln Gln Pro Tyr Thr Asp Gly Val Arg Met Glu Glu Ile Val
   545                     550                     555                     560
   Glu Gly Cys Thr Gly Ala Leu His Ile Leu Ala Arg Asp Pro Met Asn
                   565                     570                     575
   Arg Met Glu Ile Phe Arg Leu Asn Thr Ile Pro Leu Phe Val Gln Leu
                       580                     585                     590
   Leu Tyr Ser Ser Val Glu Asn Ile Gln Arg Val Ala Ala Gly Val Leu
               595                     600                     605
   Cys Glu Leu Ala Gln Asp Lys Glu Ala Ala Asp Ala Ile Asp Ala Glu
           610                     615                     620
   Gly Ala Ser Ala Pro Leu Met Glu Leu Leu His Ser Arg Asn Glu Gly
   625                     630                     635                     640
   Thr Ala Thr Tyr Ala Ala Ala Val Leu Phe Arg Ile Ser Glu Asp Lys
                   645                     650                     655
   Asn Pro Asp Tyr Arg Lys Arg Val Ser Val Glu Leu Thr Asn Ser Leu
                       660                     665                     670
   Phe Lys His Asp Pro Ala Ala Trp Glu Ala Ala Gln Ser Met Ile Pro
               675                     680                     685
   Ile Asn Glu Pro Tyr Gly Asp Asp Met Asp Ala Thr Tyr Arg Pro Met
           690                     695                     700
   Tyr Ser Ser Asp Val Pro Leu Asp Pro Leu Glu Met His Met Asp Met
   705                     710                     715                     720
   Asp Gly Asp Tyr Pro Ile Asp Thr Tyr Ser Asp Gly Leu Arg Pro Pro
                   725                     730                     735
   Tyr Pro Thr Ala Asp His Met Leu Ala
                       740                     745
<210> 5
   <211> 24
   <212> DNA
   <213> Artificial Sequence
<220>
   <223> oligonucleotide
<400> 5
   agggtctgct actgagatgc tctg 24
<210> 6
   <211> 24
   <212> DNA
   <213> Artificial sequence
<220>
   <223> oligonucleotide
<400> 6
   caaccactgg tttttctgcc accg 24
<210> 7
   <211> 22
   <212> DNA
   <213> Artificial sequence
<220>
   <223> oligonucleotide
<400> 7
   ggtgaaggtc ggtgtgaacg ga 22
<210> 8
   <211> 22
   <212> DNA
   <213> Artificial Sequence
<220>
   <223> oligonucleotide
<400> 8
   tgttagtggg gtctcgctcc tg 22
<210> 9
   <211> 24
   <212> DNA
   <213> Artificial Sequence
<220>
   <223> oligonucleotide
<400> 9
   ggcgttctct ttggaaaggt gttc 24
<210> 10
   <211> 20
   <212> DNA
   <213> Artificial Sequence
<220>
   <223> oligonucleotide
<400> 10
   ctcgaaccac atccttctct 20
<210> 11
   <211> 20
   <212> DNA
   <213> Artificial sequence
<220>
   <223> oligonucleotide
<400> 11
   gtcttatcga tgctggagtg 20
<210> 12
   <211> 20
   <212> DNA
   <213> Artificial sequence
<220>
   <223> oligonucleotide
<400> 12
   aaagctcttc tcgcagccat 20
<210> 13
   <211> 20
   <212> DNA
   <213> Artificial Sequence
<220>
   <223> oligonucleotide
<400> 13
   gcatgtccta ctcgcagcag 20
<210> 14
   <211> 20
   <212> DNA
   <213> Artificial sequence
<220>
   <223> Oligonucleotide
<400> 14
   gctgatcatg tcccggaggt 20
<210> 15
   <211> 21
   <212> DNA
   <213> Artificial Sequence
<220>
   <223> Oligonucleotide
<400> 15
   accaacagca actatgacct c 21
<210> 16
   <211> 20
   <212> DNA
   <213> Artificial Sequence
<220>
   <223> oligonucleotide
<400> 16
   aaggacgtag cgaccgcaac 20
<210> 17
   <211> 21
   <212> DNA
   <213> Artificial sequence
<220>
   <223> oligonucleotide
<400> 17
   tgcttatgga tcccagagtg a 21
<210> 18
   <211> 21
   <212> DNA
   <213> Artificial sequence
<220>
   <223> oligonucleotide
<400> 18
   ttggtgagga tctctccgcg t 21